# EUROPEAN PATENT APPLICATION

(11) **EP 2 457 915 A1**
(43) Date of publication of application: **30.05.2012**
(21) Application number: 10802364.9
(22) Date of filing: 21.07.2010
(51) Int. Cl.: C07D 491/107, C07D 498/10, G02B 5/23

(54) **CHROMENE COMPOUND**

(30) Priority: 21.07.2009 JP 2009169743
(71) Applicant: Tokuyama Corporation, Shunan-shi, Yamaguchi 745-0053 (JP)
(72) Inventor: TAKAHASHI Toshiaki, Shunan-shi Yamaguchi 745-0053 (JP); TAKENAKA Junji, Shunan-shi Yamaguchi 745-0053 (JP)
(74) Representative: Adam, Holger
(86) International application number: PCT/JP2010/062653
(87) International publication number: WO 2011/010744

(57) **Abstract**

A chromene compound having a skeleton represented by the following formula (1) and exhibiting double peak characteristic: 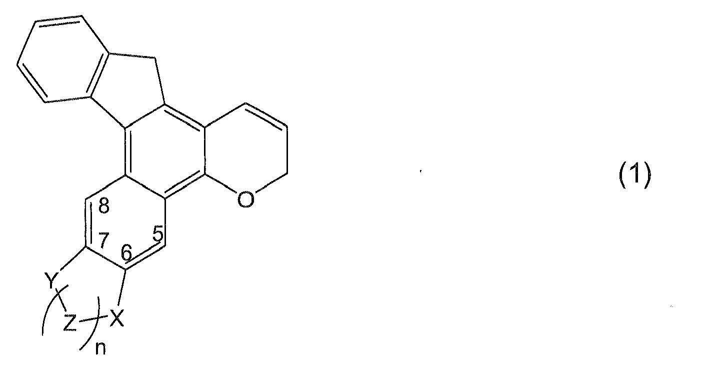 wherein Z is a group represented by any one of the following formulas: 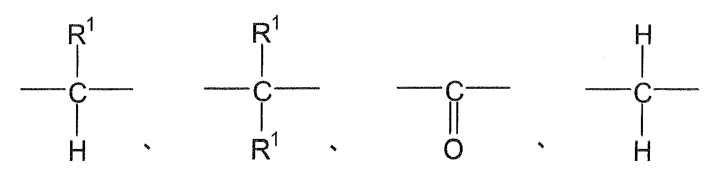 n is an integer of 1 to 3, when n is 2 or 3, Z's may be the same or different, with the proviso that when n is 1, Z cannot be -CH₂-, and when n is 2 or 3, Z's cannot be -CH₂- at the same time; R¹ is an electron absorbing group having a Hammett constant σₚ of more than 0, with the proviso that when there are a plurality of R¹'s, R¹'s may be the same or different; and X and Y are each independently an oxygen atom or =NR², with the proviso that X and Y cannot be oxygen atoms at the same time.

## Description

### TECHNICAL FIELD

The present invention relates to a novel chromene compound, a precursor thereof and use of the chromene compound.

### BACKGROUND ART

Photochromism is the reversible function of a certain compound that it changes its color swiftly upon exposure to light including ultraviolet light such as sunlight or light from a mercury lamp and returns to its original color when it is put in the dark by stopping its exposure to light. A compound having this property is called "photochromic compound" and used as a material for photochromic plastic lenses which are photochromic optical articles.

The following properties are required for the photochromic compound used for the above purpose:
(i) the degree of coloration at a visible light range before ultraviolet light is applied (to be referred to as "initial coloration" hereinafter) should be low;
(ii) the degree of coloration upon exposure to ultraviolet light (to be referred to as "color optical density" hereinafter) should be high;
(iii) the speed from the time when the application of ultraviolet light is started to the time when the color optical density reaches saturation (to be referred to as "color development sensitivity" hereinafter) should be high;
(iv) the speed from the stoppage of the application of ultraviolet light to the time when the photochromic compound returns to its original state (to be referred to as "fading speed" hereinafter) should be high;
(v) the repeat durability of this reversible function should be high; and
(vi) the solubility in a monomer composition which will become a host material after curing of the photochromic compound should be high so that its dispersibility in the host material in use becomes high.

It has been desired that the photochromic plastic lenses develop a color of a neutral tint such as grey or brown. What color is developed depends on the photochromic compound as a matter of course, which is a very important factor. When the color is to be adjusted by mixing together a plurality of photochromic compounds, there occur various problems such as a color change at the time of color development and fading (to be referred to as "color shift" hereinafter) due to the different characteristic properties of the photochromic compounds that are mixed together and a color change at the time of deterioration due to the difference in durability. To solve the above problems, a photochromic compound which by itself has two absorption wavelengths at a visible range when developing a color to develop a color of a neutral tint (to be referred to as "double peak compound" hereinafter) is important.

As the double peak compound, there are known a chromene compound represented by the following formula (A) (refer to a pamphlet of International Laid-Open WO2001/19813), a chromene compound represented by the following formula (B) or (C) (refer to a pamphlet of International Laid-Open WO2003/025638), a chromene compound represented by the following formula (D) (refer to a pamphlet of International Laid-Open WO2003/044022) and a chromene compound represented by the following formula (E) (refer to a pamphlet of International Laid-Open WO2005/028465).

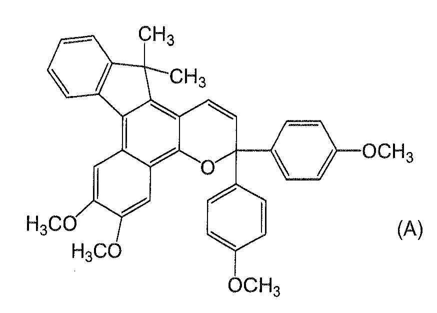

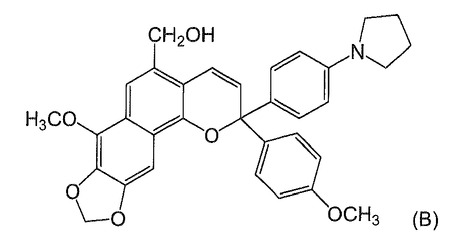

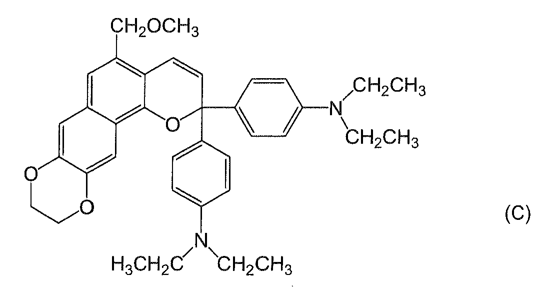

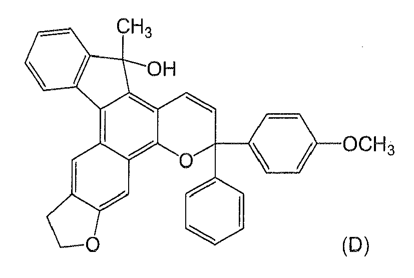

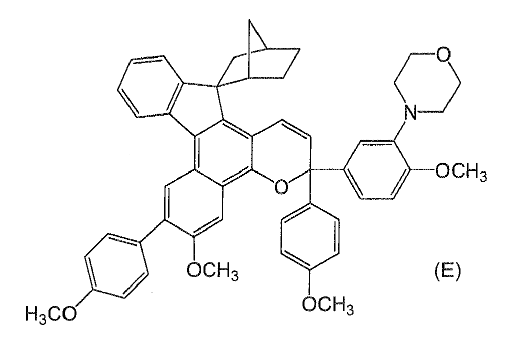

### DISCLOSURE OF THE INVENTION

In the field of photochromic plastic lenses, the requirements for photochromic properties, especially high fading speed when a photochromic plastic lens moves from outdoors to indoors and transparency when a user wears a photochromic plastic lens indoors (little initial coloration) are becoming stronger and stronger each year. Therefore, the development of a photochromic compound which satisfies all the above requirements (i) to (vi) at a higher level than the chromene compounds of the prior art is desired. When the color is to be adjusted by mixing together a plurality of photochromic compounds, it is known that a photochromic compound which develops a yellow color is generally inferior in durability to a photochromic compound which develops another color, for example, a blue color. Therefore, a compound which has a higher yellow color optical density (having a maximum absorption wavelength at 430 to 530 nm) than a blue color optical density (having a maximum absorption wavelength at 550 to 650 nm) is desired as the double peak compound (in the double peak compound, the ratio of the yellow color optical density to the blue color optical density may be referred to as "double peak characteristic" hereinafter). In consideration of these characteristic properties, the chromene compounds of the prior art have room for the improvement of the following points.

For example, although the chromene compound represented by the above formula (A) has practical levels of color optical density and double peak characteristic, it has room for improvement as it has a low fading speed. The chromene compounds represented by the above formula (B), (C) and (D) also have room for improvement as they do not have satisfactory double peak characteristic. Further, although the chromene compound represented by the above formula (E) is excellent in double peak characteristic and has practical levels of color optical density and fading speed as it is a compound whose 7-position carbon atom is substituted by a specific aryl group, it has room for improvement as the end portion of its absorption spectrum (to be referred to as "absorption end" hereinafter) goes beyond 420 nm into the visible range with the result of large initial coloration.

Therefore, it is an object of the present invention to provide a novel photochromic compound (chromene compound) which has little initial coloration, high color optical density when it is exposed to light, high color development sensitivity, high fading speed and high durability and exhibits excellent double peak characteristic.

It is another obj ect of the present invention to provide a naphthol compound which is an intermediate for the production of the chromene compound of the present invention.

Other objects and advantages of the present invention will become apparent from the following description.

The inventors of the present invention conducted intensive studies to attain the above objects and found the following fact. Although the double peak characteristic of a chromene compound having an indenonaphthopyran skeleton can be enhanced by increasing the electron donating abilities of the 6-position and 7-position substituents, when the electron donating abilities of the 6-position and 7-position substituents are increased, the fading speed becomes lower, the initial coloration becomes larger and the durability becomes lower in proportion to this.

Then, the inventors thought if the above disadvantage could be improved by adjusting the electron donating abilities of the 6-position and 7-position substituents while retaining the above advantage and investigated the introduction of various substituents.

They made further intensive studies and found that the above objects can be attained by a chromene compound having an indenonaphthopyran skeleton prepared by introducing a hetero ring condensed to the 6-position and the 7-position and further introducing an electron absorbing group onto the hetero ring. The present invention was accomplished based on this finding.

That is, firstly, the present invention is a chromene compound having a skeleton represented by the following formula (1) : 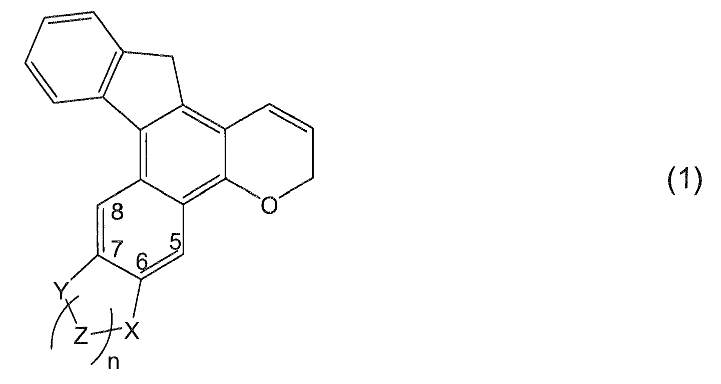 wherein Z is a group represented by any one of the following formulas: 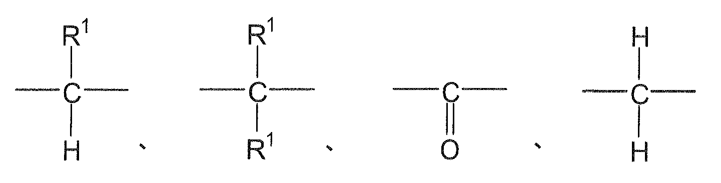 n is an integer of 1 to 3, when n is 2 or 3, Z's may be the same or different, with the proviso that when n is 1, Z cannot be -CH₂- and when n is 2 or 3, Z's cannot be -CH₂- at the same time; R¹ is an electron absorbing group having a Hammett constant σₚ of more than 0, with the proviso that when there are a plurality of R¹'s, R¹'s may be the same or different; and X and Y are each independently an oxygen atom or a group represented by the following formula (2): 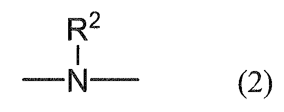 wherein R² is a hydrogen atom, hydroxyl group, alkyl group, haloalkyl group, alkenyl group, alkynyl group, cycloalkyl group, alkoxy group, aralkyl group, aralkoxy group, aryloxy group, aryl group, amino group, heterocyclic group having a nitrogen atom which is a cyclic membered hetero atom and is bonded to the nitrogen atom in the above formula (2), cyano group, nitro group, formyl group, hydroxycarbonyl group, alkylcarbonyl group, alkoxycarbonyl group or halogen atom, with the proviso that X and Y cannot be oxygen atoms at the same time.

Secondly, the present invention is a photochromic curable composition which comprises a chromene compound having a skeleton represented by the above formula (1) and a polymerizable monomer.

Thirdly, the present invention is a photochromic optical article which has a polymer molded product containing a chromene compound having a skeleton represented by the above formula (1) dispersed therein as a constituent member.

In the fourth place, the present invention is an optical article comprising an optical substrate having a surface at least part of which is coated with a polymer film as a constituent part, wherein the polymer film contains a chromene compound having a skeleton represented by the above formula (1) dispersed therein.

Finally, the present invention is a naphthol compound represented by the following formula (4): 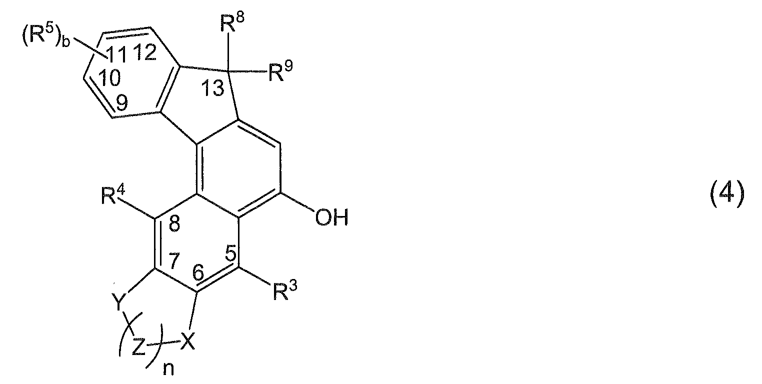 wherein R³, R⁴, R⁵, R⁸, R⁹, Z, X, Y, b and n are as defined in the above formula (3) in claim 2.

### BEST MODE FOR CARRYING OUT THE INVENTION

The chromene compound of the present invention is a chromene compound having a skeleton represented by the following formula (1). The substituents in the formula (1) will be described hereinbelow.

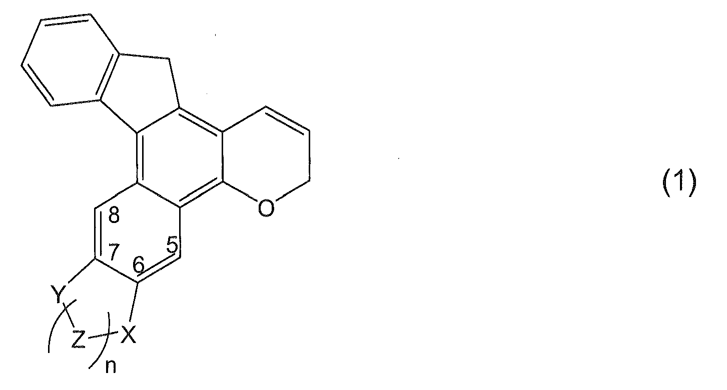

### (group Z)

The group Z is a group represented by any one of the following formulas.

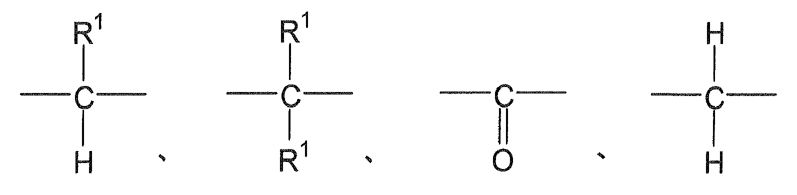

"n" which indicates the number of the groups Z is an integer of 1 to 3 . When "n" is 2 or 3, Z's may be the same or different. When "n" is 1, Z cannot be -CH₂- and when "n" is 2 or 3, Z's cannot be -CH₂- at the same time. That is, the group 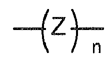 cannot be a methylene group, ethylene group and trimethylene group. Therefore, when "n" is 1, the group Z is selected from the above groups except for methylene group. When "n" is 2 or 3, at least one of Z's is selected from the above groups except for methylene group.

"n" is preferably 1 or 2 from the viewpoints of double peak characteristic and fading speed.

In the present invention, a combination of groups X, Y and Z is very important. A description is subsequently given of the group R¹ in the group Z.

### (group R¹)

The group R¹ in the group Z is an electron absorbing group having a Hammett constant σₚ of more than 0.

The Hammett constant σₚ is defined based on the Hammett equation that quantifies the electric effect of a substituent bonded to an π electron system on the basis of the dissociation constant Ka of p-substituted benzoic acid. A substituent having a Hammett constant σₚ of 0 is a hydrogen atom, and a substituent having a Hammett constant σₚ of more than 0 is a substituent having higher electron absorbing ability than the hydrogen atom. When the group Z has this group R¹, the chromene compound of the present invention exhibits an excellent effect.

Examples of the electron absorbing substituent R¹ having a σₚ of more than 0 include haloalkyl group, alkenyl group, alkynyl group, haloaryl group, cyano group (σₚ = 0.66), nitro group (σₚ = 0.78), formyl group (σₚ = 0.43), hydroxycarbonyl group (σₚ = 0.45), alkylcarbonyl group, alkoxycarbonyl group and halogen atom.

A detailed description is subsequently given of the above electron absorbing group having a Hammett constant σₚ of more than 0, that is, a positive value.

The haloalkyl group has a σₚ of 0.4 to 0.6 and is preferably a haloalkyl group having 1 to 8 carbon atoms. Preferred examples of the haloalkyl group include trifluoromethyl group (σₚ = 0.54) and 2,2,2-trifluoroethyl group.

The alkenyl group has a σₚ of more than 0 and is preferably an alkenyl group having 2 to 9 carbon atoms. Preferred examples of the alkenyl group include allyl group, propenyl group, 1-butenyl group and 2-butenyl group.

The alkynyl group has a σₚ of 0.1 to 0.3 and is preferably an alkynyl group having 2 to 9 carbon atoms. Preferred examples of the alkynyl group include propargyl group (σₚ -. 0.23) and 1-pentynyl group.

The haloaryl group has a σₚ of more than 0 and is preferably a haloaryl group having 6 to 14 carbon atoms. Preferred examples of the haloaryl group include 4-fluorophenyl group.

The alkylcarbonyl group has a σₚ of 0.4 to 0.6 and is preferably an alkylcarbonyl group having 2 to 9 carbon atoms. Preferred examples of the alkylcarbonyl group include methylcarbonyl group (σₚ = 0.50) and ethylcarbonyl group.

The alkoxycarbonyl group has a σₚ of 0.3 to 0.6 and is preferably an alkoxycarbonyl group having 2 to 9 carbon atoms. Preferred examples of the alkoxycarbonyl group include methoxycarbonyl group (σₚ = 0.45) and ethoxycarbonyl group.

Preferred examples of the halogen atom are fluorine atom (σₚ = 0.06), chlorine atom (σₚ = 0.23), bromine atom (σₚ =0.23) and iodine atom (σₚ = 0.18).

### (preferred group Z)

In the present invention, to obtain a chromene compound which has high fading speed, little initial coloration and high durability while retaining high double peak characteristic, in the group Z, the group R¹ is preferably an electron absorbing group having a σₚ of 0.05 or more and less than 0.7. Preferred examples of the group R¹ include haloalkyl group, alkynyl group, cyano group, formyl group, hydroxycarbonyl group, alkylcarbonyl group, alkoxycarbonyl group and halogen atom. From the same viewpoints, the group Z is preferably a carbonyl group.

Particularly from the viewpoint of balance between double peak characteristic and fading speed, in the group Z, the group R¹ is particularly preferably a haloalkyl group, cyano group or halogen atom, as exemplified by trifluoromethyl group, cyano group and fluorine atom. For the same reason, the group Z is preferably a carbonyl group.

### (X and Y)

In the above formula (1), X and Y are each an oxygen atom or a group represented by the following formula (2), bonded to the 6-position and 7-position carbon atoms, respectively. 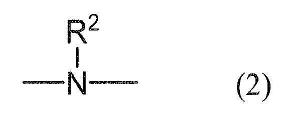 A Chromene compound in which X and Y are oxygen atoms at the same time does not exhibit high double peak characteristic. Therefore, in the formula (1), X and Y cannot be oxygen atoms at the same time.

In the above formula (2), the group R² is a hydrogen atom, hydroxyl group, alkyl group, haloalkyl group, alkenyl group, alkynyl group, cycloalkyl group, alkoxy group, aralkyl group, aralkoxy group, aryloxy group, aryl group, amino group, heterocyclic group having a nitrogen atom which is a cyclic membered hetero atom and is bonded to the nitrogen atom in the above formula (2), cyano group, nitro group, formyl group, hydroxycarbonyl group, alkylcarbonyl group, alkoxycarbonyl group or halogen atom.

The alkyl group is preferably an alkyl group having 1 to 8 carbon atoms. Preferred examples of the alkyl group include methyl group, ethyl group, n-propyl group, isopropyl group, n-butyl group, sec-butyl group, tert-butyl group, pentyl group, hexyl group, heptyl group and octyl group.

The haloalkyl group is preferably a haloalkyl group having 1 to 8 carbon atoms, as exemplified by trifluoromethyl group and 2,2,2-trifluoroethyl group.

The alkenyl group is preferably an alkenyl group having 2 to 9 carbon atoms, as exemplified by allyl group, propenyl group, 1-butenyl group and 2-butenyl group.

The alkynyl group is preferably an alkynyl group having 2 to 9 carbon atoms, as exemplified by propargyl group and 1-pentynyl group.

The cycloalkyl group is preferably a cycloalkyl group having 3 to 8 carbon atoms. Preferred examples of the cycloalkyl group include cyclopropyl group, cyclobutyl group, cyclopentyl group, cyclohexyl group, cycloheptyl group and cyclooctyl group.

The alkoxy group is preferably an alkoxy group having 1 to 8 carbon atoms. Preferred examples of the alkoxy group include methoxy group, ethoxy group, n-propoxy group, isopropoxy group, n-butoxy group, sec-butoxy group and tert-butoxy group.

The aralkyl group is preferably an aralkyl group having 7 to 11 carbon atoms. Preferred examples of the aralkyl group include benzyl group, phenylethyl group, phenylpropyl group, phenylbutyl group and naphthylmethyl group. The aralkyl group may be obtained by substituting one or more hydrogen atoms on a benzene ring by the above alkyl group, cycloalkyl group, alkoxy group, aralkyl group or aryloxy group.

The aralkoxy group is preferably an aralkoxy group having 7 to 11 carbon atoms. Preferred examples of the aralkoxy group include benzyloxy group and naphthylmethoxy group.

The aryloxy group is preferably an aryloxy group having 7 to 11 carbon atoms. Preferred examples of the aryloxy group include phenyloxy group and naphthyloxy group. The aryloxy group may be obtained by substituting one or more hydrogen atoms on a benzene ring by the above alkyl group, cycloalkyl group, alkoxy group, aralkyl group or aryloxy group.

The aryl group is preferably an aryl group having 6 to 14 carbon atoms. Preferred examples of the aryl group include phenyl group and 1-naphthyl group. The aryl group may be obtained by substituting one or more hydrogen atoms on a benzene ring by the above alkyl group, cycloalkyl group, alkoxy group, aralkyl group or aryloxy group.

The amino group is not limited to a primary amino group and may be a secondary amino group or tertiary amino group having a substituent. The typical substituent of the amino group is an alkyl group or an aryl group. Preferred examples of the substituted amino group (secondary amino group or tertiary amino group) include alkylamino groups such as methylamino group and ethylamino group; dialkylamino groups such as dimethylamino group and diethylamino group; arylamino groups such as phenylamino group; and diarylamino groups such as diphenylamino group.

Examples of the heterocyclic group having a nitrogen atom as a member hetero atom and bonded to the nitrogen atom in the formula (2) via the above nitrogen atom include morpholino group, piperidino group, pyrrolidinyl group, piperazino group, N-methylpiperazino group and indolinyl group. Further, these heterocyclic groups may have a substituent. Examples of the substituent include alkyl groups such as methyl group. Examples of the heterocyclic group having a substituent include 2,6-dimethylmorpholino group, 2,6-dimethylpiperidino group and 2,2,6,6-tetramethylpiperidino group.

The alkylcarbonyl group is preferably an alkylcarbonyl group having 2 to 9 carbon atoms, as exemplified by methylcarbonyl group and ethylcarbonyl group.

The alkoxycarbonyl group is preferably an alkoxycarbonyl group having 2 to 9 carbon atoms, as exemplified by methoxycarbonyl group and ethoxycarbonyl group.

Examples of the halogen atom are fluorine atom, chlorine atom, bromine atom and iodine atom.

Out of the groups represented by the above formula (2), the group R² is preferably a group having a σₚ of -0.20 to 1.00 from the viewpoints of double peak characteristic and fading speed. More specifically, the group R² is preferably a hydrogen atom, alkyl group, haloalkyl group, cyano group, nitro group or halogen atom. From the viewpoint of double peak characteristic, it is particularly preferably a group having a σₚ of -0.20 to 0.3, as exemplified by hydrogen atom, alkyl group and halogen atom. The particularly preferred substituent is a hydrogen atom, methyl group or fluorine atom.

Particularly preferred combinations of Z, n, X and Y are given below. In the following formulas, carbon atoms at positions denoted by 6 and 7 are the 6-position and the 7-position carbon atoms in the above formula (1), respectively.

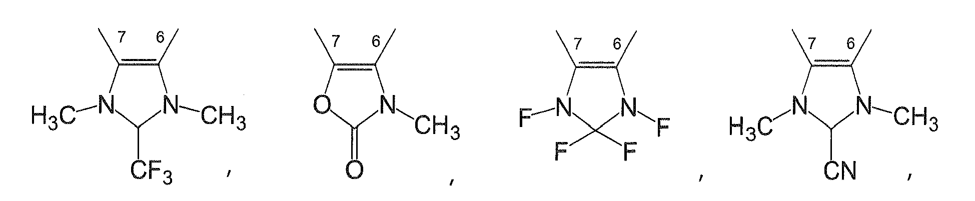

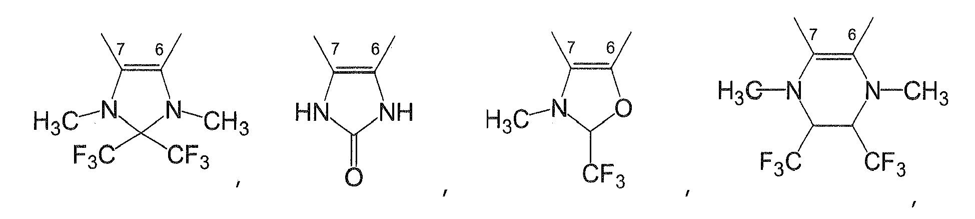

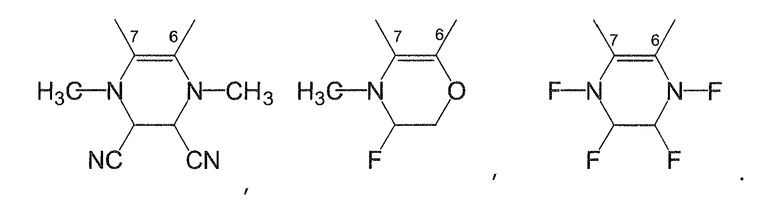

When the chromene compound of the present invention has an indenonaphthopyran skeleton as represented by the above formula (1) and the above 6-position and 7-position substituents as described above, it exhibits excellent photochromic properties. Therefore, other groups are not particularly limited. However, a chromene compound which exhibits particularly excellent photochromic properties is preferably a chromene compound in which other groups are specified as follows. A description is subsequently given of this preferred chromene compound.

### (preferred chromene compound)

In the present invention, out of the chromene compounds having a skeleton represented by the above formula (1), a chromene compound represented by the following formula (3) is preferred.

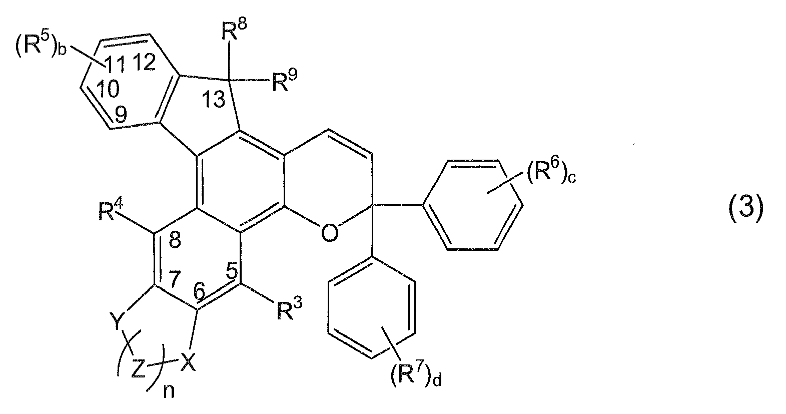

In the above formula (3), Z, n, X and Y are as defined in the above formula (1). A description is subsequently given of groups other than these groups.

### (groups R³ and R⁴)

In the chromene compound represented by the above formula (3), the 5-position group R³ and the 8-position group R⁴ are each independently a hydrogen atom, hydroxyl group, alkyl group, haloalkyl group, alkenyl group, alkynyl group, cycloalkyl group, alkoxy group, aralkyl group, aralkoxy group, aryloxy group, aryl group, amino group, heterocyclic group having a nitrogen atom as a member hetero atom and bonded to the 5-position or 8-position carbon atom via the nitrogen atom, cyano group, nitro group, formyl group, hydroxycarbonyl group, alkylcarbonyl group, alkoxycarbonyl group or halogen atom. Preferred examples of the above alkyl group, haloalkyl group, alkenyl group, alkynyl group, cycloalkyl group, alkoxy group, aralkyl group, aralkoxy group, aryloxy group, aryl group, amino group, heterocyclic group having a nitrogen atom as a member hetero atom and bonded to the 5-position or 8-position carbon atom via the nitrogen atom, alkylcarbonyl group, alkoxycarbonyl group and halogen atom are the same as those enumerated for the above group R².

In the present invention, the groups R³ and R⁴ are involved in fading speed. To accelerate the fading speed, R³ preferably has a stereoscopically small substituent. To this end, the group R³ is particularly preferably a hydrogen atom.

### (group R⁵)

In the chromene compound represented by the above formula (3), the 9- to 12-position substituent R⁵ is a hydroxyl group, alkyl group, haloalkyl group, alkenyl group, alkynyl group, cycloalkyl group, alkoxy group, aralkyl group, aralkoxy group, aryloxy group, aryl group, amino group, heterocyclic group having a nitrogen atom as a member hetero atom and bonded to a benzene ring bonded thereto via the nitrogen atom, cyano group, nitro group, formyl group, hydroxycarbonyl group, alkylcarbonyl group, alkoxycarbonyl group or halogen atom. Preferred examples of the above alkyl group, haloalkyl group, alkenyl group, alkynyl group, cycloalkyl group, alkoxy group, aralkyl group, aralkoxy group, aryloxy group, aryl group, amino group, heterocyclic group having a nitrogen atom as a member hetero atom and bonded to a benzene ring bonded thereto via the nitrogen atom, alkylcarbonyl group, alkoxycarbonyl group and halogen atom are the same as those enumerated for the above group R².

"b" which indicates the number of R⁵'s is an integer of 0 to 4. When "b" is 2 to 4, R⁵'s may be the same or different.

In the present invention, the group R⁵ is involved in fading speed. The group R⁵ is preferably a hydrogen atom ("b" is 0) or an electron absorbing group. When the group R⁵ is an electron absorbing group, it is preferably bonded to the 11-position carbon atom so as to accelerate the fading speed. The particularly preferred electron absorbing group is a cyano group or haloalkyl group, more specifically, cyano group or trifluoromethyl group.

### (groups R⁶ and R⁷)

In the above formula (3), R⁶ and R⁷ are each independently a hydroxyl group, alkyl group, haloalkyl group, alkenyl group, alkynyl group, cycloalkyl group, alkoxy group, aralkyl group, aralkoxy group, aryloxy group, aryl group, amino group, heterocyclic group having a nitrogen atom as a member hetero atom and bonded to a benzene ring bonded thereto via the nitrogen atom, cyano group, nitro group, formyl group, hydroxycarbonyl group, alkylcarbonyl group, alkoxycarbonyl group or halogen atom. Preferred examples of the above alkyl group, haloalkyl group, alkenyl group, alkynyl group, cycloalkyl group, alkoxy group, aralkyl group, aralkoxy group, aryloxy group, aryl group, amino group, heterocyclic group having a nitrogen atom as a member hetero atom and bonded to a benzene ring bonded thereto via the nitrogen atom, alkylcarbonyl group, alkoxycarbonyl group and halogen atom are the same as those enumerated for the above group R².

"c" and "d" which indicate the numbers of the substituents R⁶ and R⁷, respectively, are each independently an integer of 0 to 5. When "c" and "d" are each 2 or more, R⁶'s and R⁷'s may be the same or different.

In the present invention, the substituents R⁶ and R⁷ are involved in double peak characteristic and fading speed. Although the numbers and positions of the substituents are not particularly limited, they are preferably existent at the p-position in order to obtain high double peak characteristic and a high fading speed. The preferred substituents are each a hydrogen atom ("b" and "c" are 0), alkyl group, alkoxy group, amino group or heterocyclic group having a nitrogen atom as a member hetero atom and bonded to a benzene bonded thereto via the nitrogen atom. Examples of these substituents include methyl group, methoxy group, dimethylamino group and morpholino group. From the viewpoint of the double peak characteristic, the substituents are each particularly preferably a hydrogen atom, alkyl group or alkoxy group, more specifically, hydrogen atom, methyl group or methoxy group.

### (groups R⁸ and R⁹)

In the chromene compound represented by the above formula (3), the 13-position groups R⁸ and R⁹ are each independently a hydrogen atom, hydroxyl group, alkyl group, haloalkyl group, alkenyl group, alkynyl group, cycloalkyl group, alkoxy group, aralkyl group, aralkoxy group, aryloxy group, aryl group, amino group, heterocyclic group having a nitrogen atom as a member hetero atom and bonded to the 13-position carbon atom via the nitrogen atom, cyano group, nitro group, formyl group, hydroxycarbonyl group, alkylcarbonyl group, alkoxycarbonyl group or halogen atom.

Preferred examples of the above alkyl group, haloalkyl group, alkenyl group, alkynyl group, cycloalkyl group, alkoxy group, aralkyl group, aralkoxy group, aryloxy group, aryl group, amino group, heterocyclic group having a nitrogen atom as a member hetero atom and bonded to the 13-position carbon atom via the nitrogen atom, alkylcarbonyl group, alkoxycarbonyl group and halogen atom are the same as those enumerated for the above group R².

R⁸ and R⁹ may be bonded together to form a carbonyl group or aliphatic hydrocarbon ring together with the 13-position carbon atom bonded thereto.

The number of member carbon atoms of the aliphatic hydrocarbon ring is preferably 4 to 20, more preferably 4 to 15 from the viewpoints of color optical density and fading speed. From the viewpoint of fading speed, the number of member carbon atoms is particularly preferably 4 to 12. This aliphatic hydrocarbon ring may have at least one substituent selected from alkyl group, haloalkyl group, cycloalkyl group, alkoxy group, amino group, aralkyl group, aryl group and halogen atom. Preferred examples of the alkyl group, haloalkyl group, cycloalkyl group, alkoxy group, amino group, aralkyl group, aralkoxy group, aryl group and halogen atom as substituents are the same as those enumerated for the group R². Out of these, an alkyl group is preferred from the viewpoints of color optical density and fading speed, as exemplified by methyl group.

In the present invention, preferred substituents R⁸ and R⁹ are each selected from alkyl group, alkoxy group and hydroxyl group, or R⁸ and R⁹ are bonded together to form an aliphatic hydrocarbon ring together with the 13-position carbon atom. An example of the alkyl group is a methyl group, and an example of the alkoxy group is a methoxy group. Out of the above preferred substituents, R⁸ and R⁹ are preferably bonded together to form an aliphatic hydrocarbon ring together with the 13-position carbon atom so as to reduce color development by heat at room temperature under no exposure to light (this color development will be referred to as "initial coloration due to thermochromism" hereinafter) and accelerate the fading speed while retaining the double peak characteristic. The aliphatic hydrocarbon ring is preferably a single ring having 4 to 20 member carbon atoms, bicyclo ring or tricyclo ring. Specific examples of the aliphatic hydrocarbon ring include single rings such as cyclobutane ring, cyclopentane ring, cyclohexane ring, cycloheptane ring, cyclooctane ring, cyclononane ring, cyclodecane ring and 3,3,5,5-tetramethylcyclohexane ring, bicyclo rings such as bicyclo[2,2,1]heptane ring, bicyclo[3,2,1]octane ring and bicyclo[3,3,1]nonane ring, and tricyclo rings such as adamantane ring.

Out of these, a single ring formed by bonding together the groups R⁸ and R⁹ exhibits a particularly excellent effect. Specific examples of the single ring include cyclobutane ring, cyclopentane ring, cyclohexane ring, cycloheptane ring, cyclooctane ring, cyclononane ring, cyclodecane ring and 3,3,5,5-tetramethylcyclohexane ring.

Out of the above single rings, cyclooctane ring and 3,3,5,5-tetramethylcyclohexane ring are particularly preferred.

### (preferred examples of chromene compound)

In the present invention, preferred examples of the chromene compound are the following compounds.

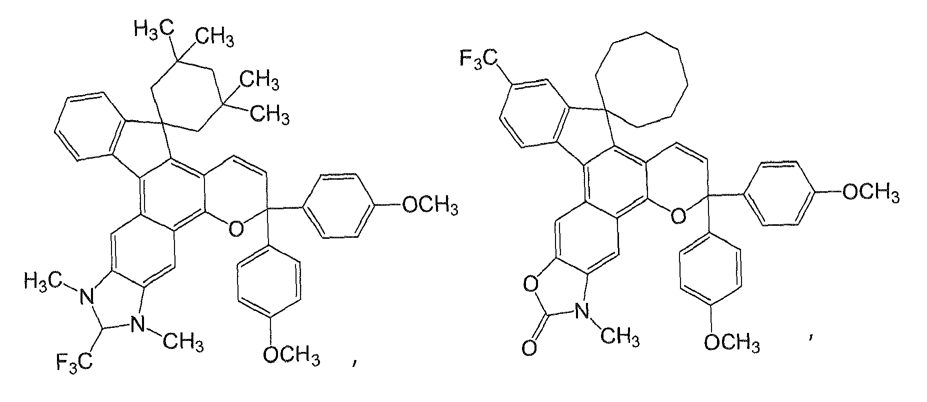

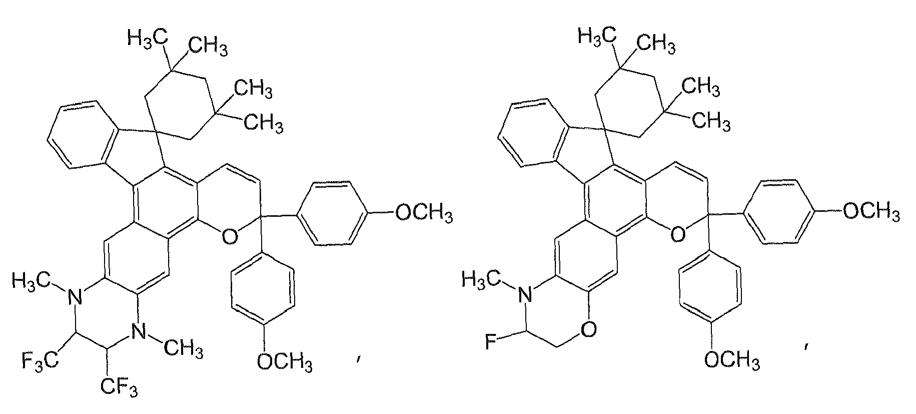

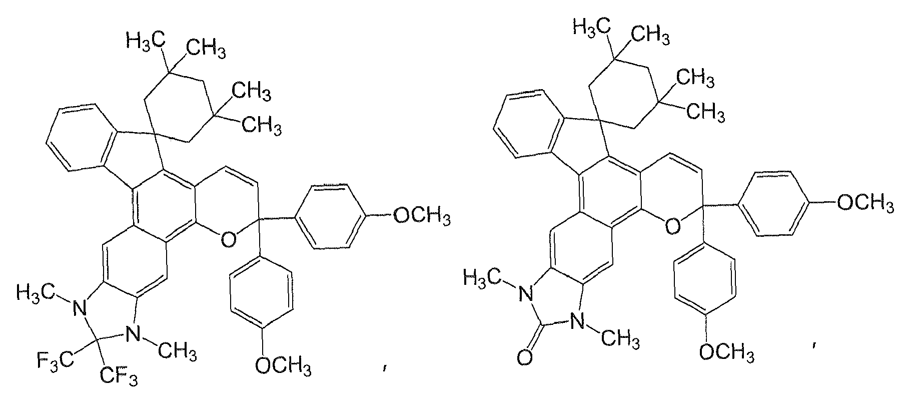

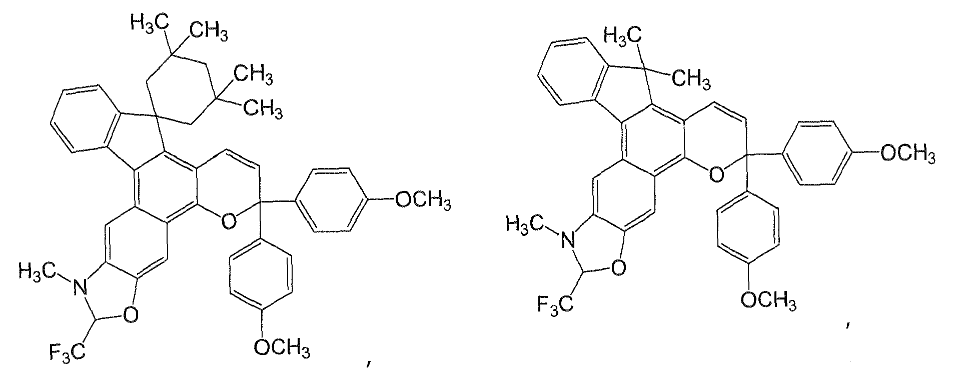

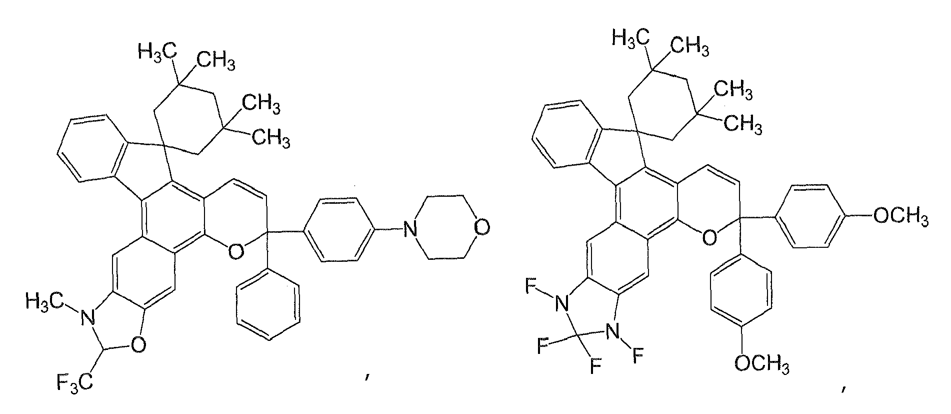

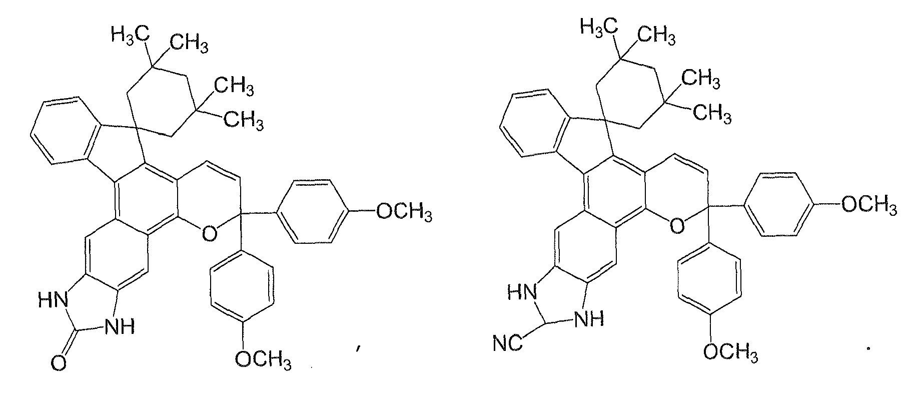

### (identification of chromene compound)

The chromene compound of the present invention is existent as an achromatic or light yellow solid or viscous liquid at normal temperature and normal pressure and can be confirmed by the following means (1) to (3).
(1) When the proton nuclear magnetic resonance spectrum (¹H-NMR) of the chromene compound is measured, a peak based on an aromatic proton appears at δ of around 5.0 to 9.0 ppm and peaks based on the protons of an alkyl group and an alkoxy group appear at δ of around 0.5 to 4.5 ppm. By comparing these spectral intensities relatively, the number of the protons of bonds can be known.
(2) The composition of a corresponding compound can be determined by elemental analysis.
(3) When the ¹³C-nuclear magnetic resonance spectrum (¹³C-NMR) of the chromene compound is measured, a peak based on the carbon of an aromatic hydrocarbon group appears at δ of around 110 to 160 ppm, and peaks based on the carbons of an alkyl group and an alkoxy group appear at δ of around 10 to 80 ppm.

### (production process of chromene compound)

The production process of the chromene compound of the present invention is not particularly limited, and any synthesis process may be employed. As a typical process which is advantageously employed, a naphthol compound and a propargyl alcohol compound are reacted with each other. The production process of the chromene compound represented by the above formula (3) will be described hereinbelow as an example.

The chromene compound represented by the above formula (3) can be advantageously produced by reacting a naphthol compound represented by the following formula (4) with a propargyl alcohol compound represented by the following formula (5) in the presence of an acid catalyst. 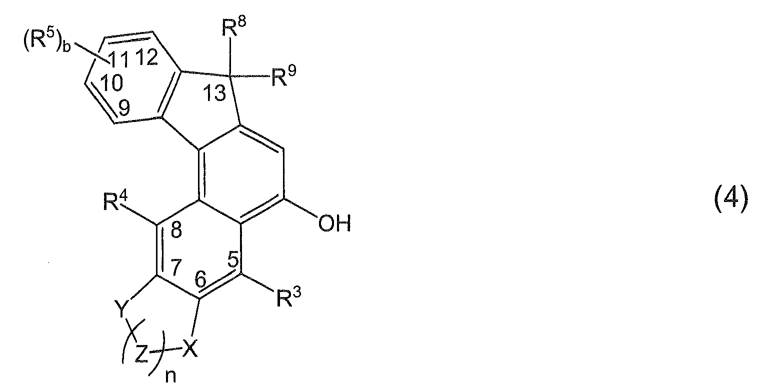 (wherein R³, R⁴, R⁵, R⁸, R⁹, Z, X, Y, b and n are as defined in the above formula (3).) 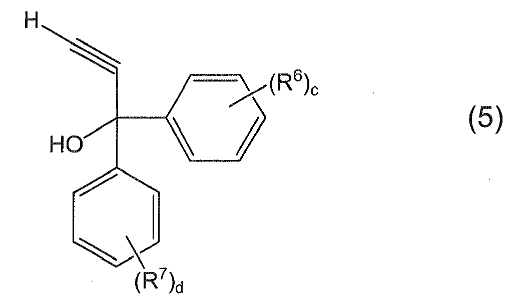 (wherein R⁶, R⁷, c and d are as defined in the above formula (3).)

The naphthol compound represented by the above formula (4) is provided as a novel compound by the present invention. In the formula (4), it should be understood that the definitions and examples of R³, R⁴, R⁵, R⁸, R⁹, Z, X, Y, "b" and "n" are the same as those in the above formula (3).

Preferred examples of the above naphthol compound are compounds represented by the following formulas.

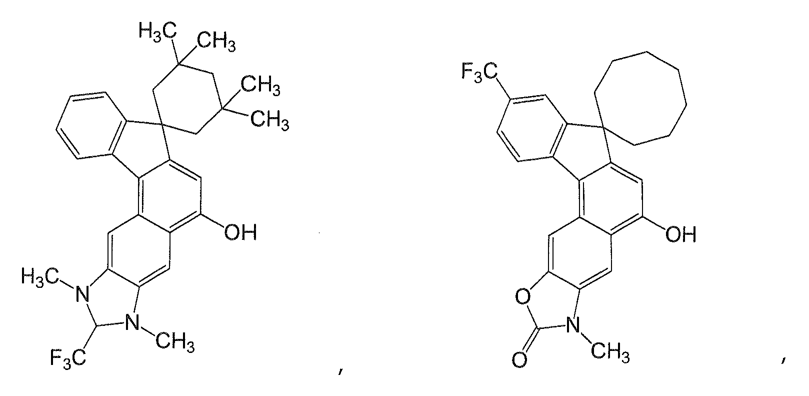

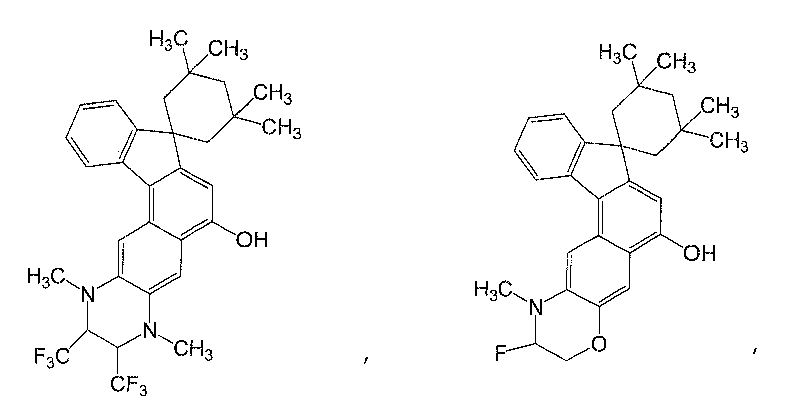

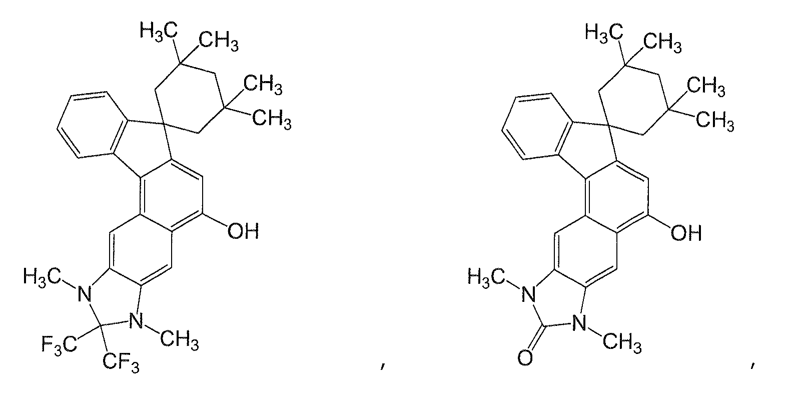

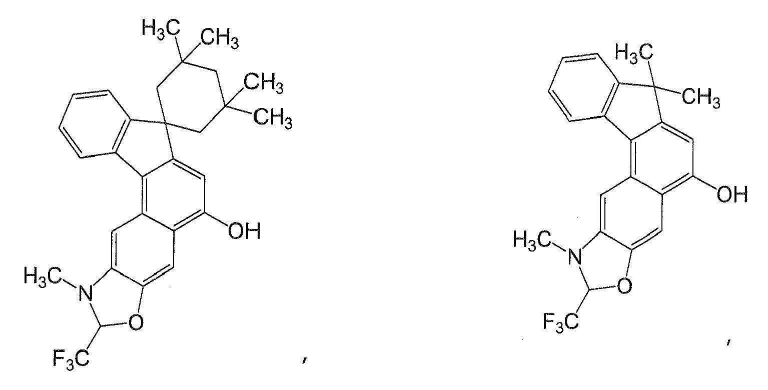

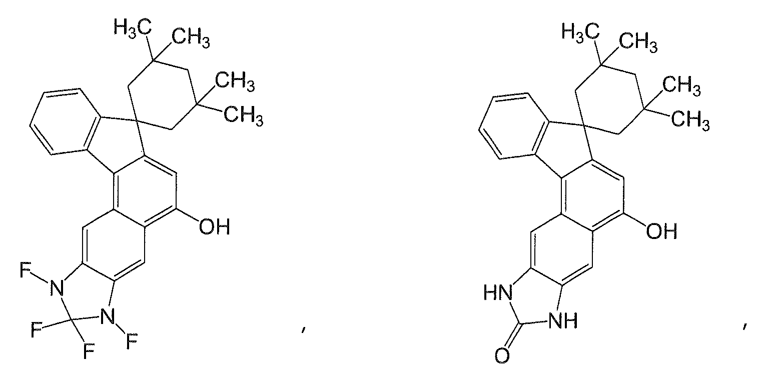

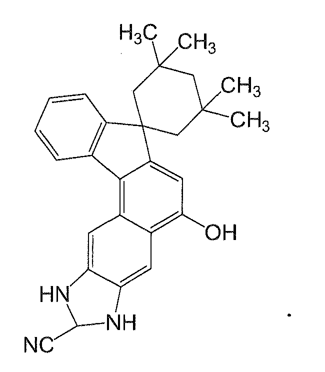

Ordinary naphthol compounds can be synthesized in accordance with a reaction method described in, for example, research papers such as International Laid-open WO2001/60881. The propargyl alcohol compound represented by the above formula (5) can be synthesized, for example, by reacting a benzophenone compound corresponding to the above formula (5) with a metal acetylene compound such as lithium acetylide.

### (process of synthetizing naphthol compound)

Although the process of synthesizing the naphthol compound represented by the above formula (4) is not particularly limited, it can be synthesized as follows, for example.

A benzene compound represented by the following formula (I) may be purchased as a commercially available product or may be synthesized based on the following documents. 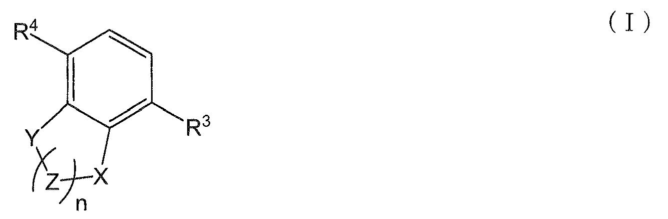 In the above formula, X, Y, Z and "n" are as defined in the above formula (1), and R³ and R⁴ are as defined in the above formula (3).

For example, a benzene compound represented by the following formula (6) can be purchased as a commercially available product.

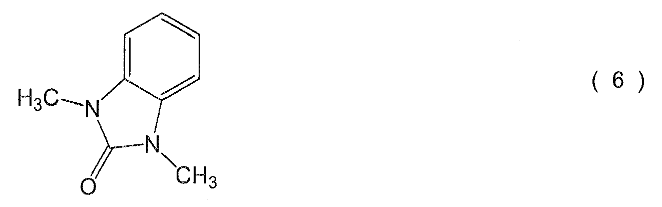

For example, a benzene compound represented by the following formula (7) can be synthesized in accordance with a reaction method described in research papers such as Synthesis.(10). 657-658; 1975.

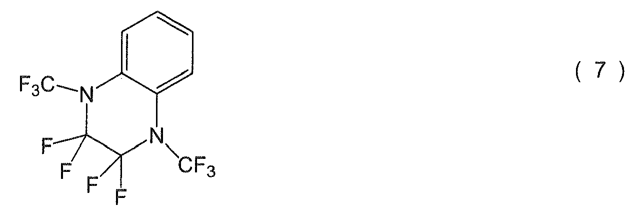

For example, a benzene compound represented by the following formula (8) can be synthesized in accordance with a reaction method described in research papers such as Hunan Daxue Xuebao, Ziran Kexueban. 28(2), 16-20. 39; 2001.

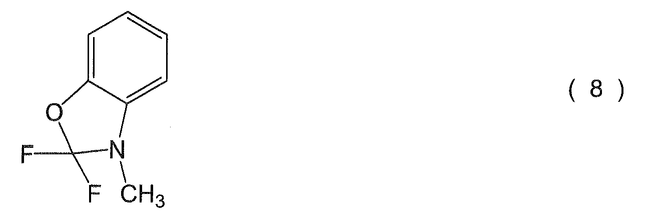

After the obtained benzene compound is brominated, a Grignard reagent is prepared and reacted with acid chloride to obtain a benzophenone compound represented by the following formula (9). 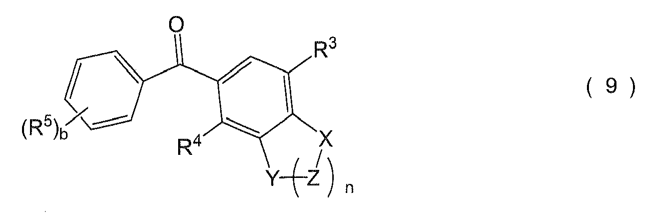 As the compound represented by the above formula (6) and having a carbonyl group is used a compound obtained by carrying out acetal protection with ethylene glycol. A naphthalene compound represented by the following formula (10) is obtained by carrying out the Stobbe reaction and cyclization reaction of the above benzophenone compound and hydrolyzed by using an alkali or acid to obtain a carboxylic acid represented by the following formula (11). 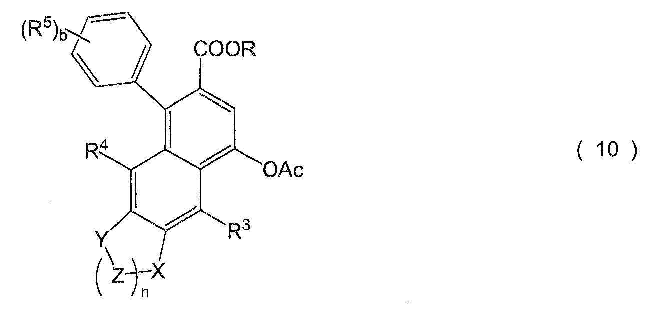 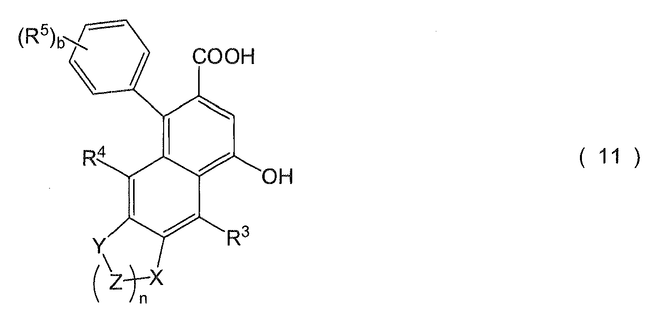 R⁵ and "b" in the above formulas (9) to (11) are as defined in the above formula (3). The carboxylic acid is benzylated by using a base such as potassium carbonate and benzyl chloride and then hydrolyzed by using an alkali or acid to obtain a carboxylic acid which is benzyl protected and represented by the following formula (12). 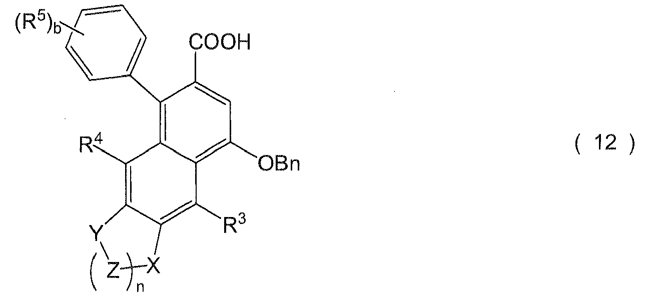 The benzyl protected carboxylic acid is converted into an amine by a method such as Curtius rearrangement, Hofmann rearrangement or Lossen rearrangement, and a diazonium salt is prepared from the amine. This diazonium salt is converted into a bromide through a Sandmeyer reaction or the like, and the obtained bromide is reacted with magnesium or lithium to prepare an organic metal reagent. This organic metal reagent is reacted with a ketone represented by the following formula (13) at -80 to 70°C in an organic solvent for 10 minutes to 4 hours to obtain an alcohol material represented by the following formula (14). 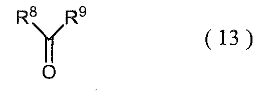 (R⁸ and R⁹ in the above formula (13) are as defined in the above formula (3).) 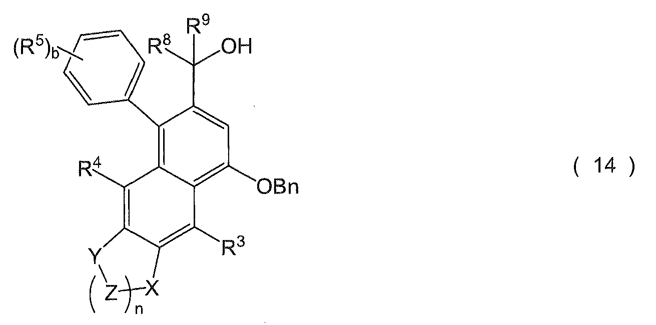 (R⁸ and R⁹ in the above formula (14) are as defined in the above formula (3).)
The debenzylation reaction of this alcohol material is carried out with hydrogen and palladium carbon and then a Friedel-Crafts reaction is carried out at 10 to 120°C for 10 minutes to 2 hours under a neutral to acid condition to synthesize a naphthol compound of interest. In the above reaction, the reaction ratio of the above organic metal reagent to the ketone represented by the above formula (13) is selected from among a wide range but generally selected from a range of 1:10 to 10:1 (molar ratio). The reaction temperature is preferably -80 to 70°C, and an aprotic organic solvent such as diethyl ether, tetrahydrofuran, benzene or toluene is used as the solvent. The naphthol compound represented by the above formula (4) can be obtained by carrying out the Friedel-Crafts reaction of the alcohol material in the neutral to acid condition. The acid catalyst is preferably selected from acetic acid, hydrochloric acid, sulfuric acid, benzenesulfonic acid, p-toluenesulfonic acid and acid alumina. The acid catalyst is preferably used in an amount of 0.1 to 10 parts by weight based on 100 parts by weight of the alcohol material. For this reaction, a solvent such as tetrahydrofuran, benzene or toluene is used.

### (identification of naphthol compound)

The naphthol compound of the present invention is existent as an achromatic or light yellow solid or viscous liquid at normal temperature and normal pressure and can be confirmed by the following means (1) to (3).
(1) When the proton nuclear magnetic resonance spectrum (¹H-NMR) of the naphthol compound is measured, a peak based on an aromatic proton appears at δ of around 5.0 to 9.0 ppm and peaks based on the protons of an alkyl group and an alkoxy group appear at δ of around 0.5 to 4.5 ppm. By comparing these spectral intensities relatively, the number of the protons of bonds can be known.
(2) The composition of a corresponding compound can be determined by elemental analysis.
(3) When the ¹³C-nuclear magnetic resonance spectrum (¹³C-NMR) of the naphthol compound is measured, a peak based on the carbon of an aromatic hydrocarbon group appears at δ of around 110 to 160 ppm, and peaks based on the carbons of an alkyl group and an alkoxy group appear at δ of around 10 to 80 ppm.

### (synthesis of propargyl alcohol)

The propargyl alcohol compound represented by the above formula (5) is an already known compound. The propargyl alcohol compound can be synthesized, for example, by reacting a benzophenone compound corresponding to the above formula (5) with a metal acetylene compound such as lithium acetylide.

### (specific production process of chromene compound)

The reaction ratio of the naphthol compound to the propargyl alcohol compound is selected from a wide range, preferably 1:10 to 10:1 (molar ratio) . As the acid catalyst is used sulfuric acid, benzenesulfonic acid, p-toluenesulfonic acid or acid alumina. The acid catalyst is used in an amount of 0.1 to 10 parts by weight based on 100 parts by weight of the total of the naphthol compound and the propargyl alcohol compound. The reaction temperature is preferably 0 to 200°C. An aprotic organic solvent such as N-methylpyrrolidone, dimethyl formamide, tetrahydrofuran, benzene or toluene is preferably used as the solvent. The method of purifying the product obtained through the above reaction is not particularly limited. For example, the obtained product may be purified by carrying out silica gel column purification and further recrystallization.

### (characteristic properties of chromene compound)

Since the chromene compound of the present invention has high double peak characteristic, when it is mixed with another photochromic compound to prepare a photochromic composition which develops a brown or grey color, the amount of the photochromic compound which develops a yellow color and has low durability can be reduced. Therefore, a color change at the time of fading and a color change at the time of deterioration hardly occur. Further, since the chromene compound of the present invention has little initial coloration, an optical article containing the chromene compound of the present invention, for example, a photochromic lens containing the chromene compound of the present invention has high transparency under no exposure to light.

The photochromic compound to be mixed with the chromene compound of the present invention so as to adjust the color is not particularly limited, and a known compound may be used. Examples of this photochromic compound include chromene compounds described in a pamphlet of International Laid-open WO2001/060811 and JP-A 2009-67680.

### (use purpose of chromene compound)

The chromene compound of the present invention exhibits excellent photochromic properties as described above. A polymer material dispersion of the chromene compound of the present invention is most practical, and a photochromic optical article having a polymer molded product containing the chromene compound of the present invention dispersed therein as a constituent member exhibits excellent photochromic properties. Therefore, the chromene compound of the present invention can be used especially in photochromic lenses (optical articles).

When the chromene compound of the present invention is used in a photochromic lens, a lens can be formed by a method known per se, capable of obtaining uniform light controllability. For example, a method in which a thermoplastic resin and the chromene compound of the present invention are mixed together in a molten state to form a lens is employed. Further, a method in which a polymer film containing the chromene compound of the present invention dispersed uniformly therein is formed on the surface of a lens, or a method in which the chromene compound of the present invention is dissolved, for example, in silicone oil and impregnated into the surface of a lens at 150 to 200°C for 10 to 60 minutes is also employed. When the chromene compound is dispersed into the surface portion of the lens as described above, the surface of the lens may be further coated with a curable substance as required to obtain a photochromic lens.

Moreover, a method in which a photochromic curable composition containing the chromene compound of the present invention and a polymerizable monomer is polymerized by a predetermined method to obtain a lens may be employed. In the method using this photochromic curable composition, a photochromic lens can be formed directly by polymerizing the curable composition by a known method. Further, the photochromic curable composition may be applied to a plastic lens (optical substrate) and polymerized and cured to form a polymer film containing the chromene compound of the present invention dispersed therein on the optical substrate, thereby obtaining a photochromic lens (optical article) (this method may be referred to as "coating method"). When the polymer film containing the chromene compound of the present invention dispersed therein is formed on the optical substrate, the surface of the polymer film may be further coated with a curable substance.

In the above photochromic curable composition, the polymerizable monomer in use is not particularly limited, known polymerizable monomers may be used, and a combination of known polymerizable monomers may be selected according to the desired performance of an optical article.

### Examples

The following examples are provided for the purpose of further illustrating the present invention but are in no way to be taken as limiting.

### Example 1

1.15 g (2.33 mmol) of a naphthol compound represented by the following formula (15) and 0.92 g (3.43 mmol) of a propargyl alcohol compound represented by the following formula (16) were dissolved in 50 ml of toluene, 0.022 g of p-toluenesulfonic acid was further added, and the obtained mixture was refluxed for 1 hour. 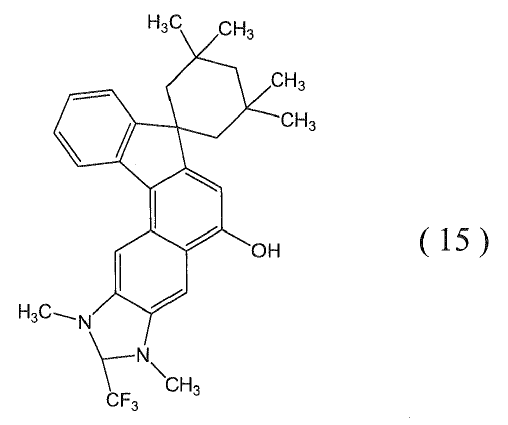 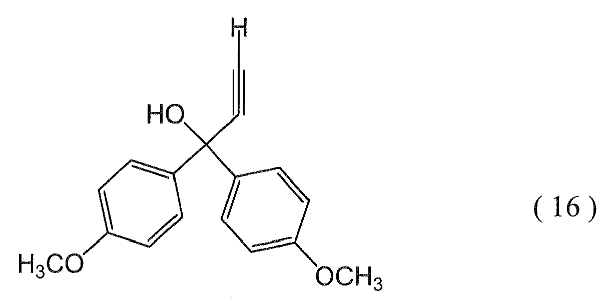 After the reaction, the solvent was removed, the obtained product was purified by column chromatography, and crystallization was carried out with methanol (5 ml) to obtain 1.21 g of a white powder (yield rate of 70 %). The elemental analysis values of this product were 75.66 % of C, 6.44 % of H and 3.71 % of N which were almost equal to the calculated values of C₄₇H₄₇F₃N₂O₃ (C: 75.78 %, H: 6.36 %, N: 3.76 %).

When the proton nuclear magnetic resonance spectrum of the product was measured, it showed 31H peaks based on an alkyl group and an alkoxy group at δ of around 0.5 to 4.5 ppm and a 16H peak based on an aromatic proton at δ of around 5.0 to 9.0 ppm.

Further, when the ¹³C-nuclear magnetic resonance spectrum was measured, it showed a peak based on the carbon of an aromatic ring at δ of around 110 to 160 ppm and peaks based on the carbons of an alkyl group and an alkoxy group at δ of around 10 to 80 ppm.

It was confirmed from the above results that the isolated product was a compound represented by the following formula (17).

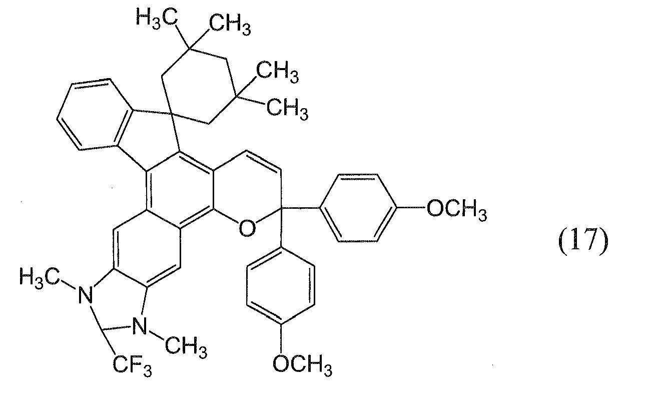

### Examples 2 and 3

Chromene compounds shown in Table 1 were synthesized in the same manner as in Example 1. When the structures of the obtained products were analyzed by using the same structure checking means as in Example 1, it was confirmed that they were compounds represented by structural formulas shown in Table 1. The elemental analysis values, calculated values obtained from the structural formulas of the compounds and characteristic ¹H-NMR spectra of these compounds are shown in Table 2.

**Table 1**

| Example No. | Raw materials | | Product | Yield rate(%) |
|---|---|---|---|---|
| | Naphthol compound | Propargyl alcohol compound | | |
| 2 | 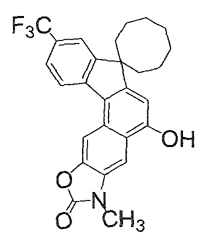 | 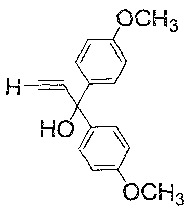 | 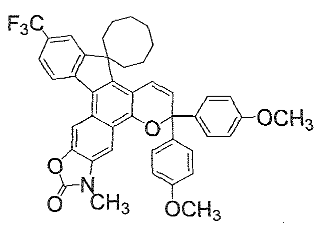 | 65 |
| 3 | 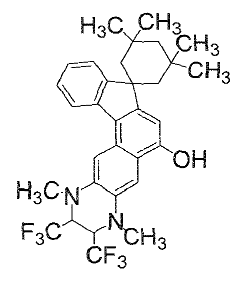 | 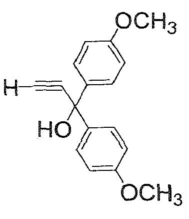 | 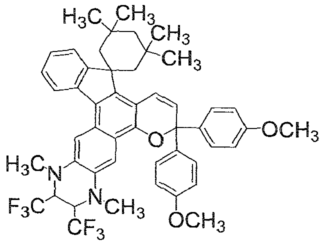 | 67 |

**Table 2**

| Example No. | Elemental analysis values | | | | | | ¹H-NMR (NMR) |
|---|---|---|---|---|---|---|---|
| | Experimental values | | | Calculated values | | | |
| | C | H | N | C | H | N | |
| 2 | 73.55 | 5.44 | 1.90 | 73.63 | 5.34 | 1.95 | δ5.0-9.0 15H |
| | | | | | | | δ0.5-4.5 23H |
| 3 | 71.27 | 5.95 | 3.28 | 71.17 | 5.85 | 3.39 | δ5.0-9.0 16H |
| | | | | | | | δ0.5-4.5 32H |

### Example 4

### (evaluation of physical properties of photochromic plastic lens produced by coating method)

Each of the chromene compounds obtained in the above examples was mixed with a photopolymerization initiator and a polymerizable monomer, the resulting mixture was applied to the surface of a lens substrate, and ultraviolet light was applied to polymerize the coating film on the surface of the lens substrate.

A photochromic curable composition prepared by mixing together 50 parts by mass of 2,2-bis(4-methacryloyloxypentaethoxyphenyl)propane, 10 parts by mass of polyethylene glycol diacrylate (average molecular weight of 532), 10 parts by mass of trimethylolpropane trimethacrylate, 10 parts by mass of polyester oligomer hexaacrylate (EB-1830 of Daicel UCB Co. , Ltd.) and 10 parts by mass of glycidyl methacrylate as radical polymerizable monomers was used. 1 part by mass of the chromene compound obtained in Example 1 was added to and fully mixed with 90 parts by mass of this mixture of the radical polymerizable monomers, and 0.3 part by mass of CGI1800 {a mixture of 1-hydroxycyclohexylphenyl ketone and bis(2,6-dimethoxybenzoyl)-2,4,4-trimethyl-pentyl phosphine oxide (weight ratio of 3:1)} as a photopolymerization initiator, 5 parts by mass of bis(1,2,2,6,6-pentamethyl-4-piperidyl)sebacate, 3 parts by mass of ethylenebis(oxyethylene)bis[3-(5-tert-butyl-4-hydroxy-m-tolyl)propionate] as a stabilizer, 7 parts by mass of γ-methacryloyloxypropyl trimethoxysilane as a silane coupling agent and 3 parts by mass of N-methyldiethanolamine were added to and fully mixed with the above mixture to obtain a photochromic curable composition.

Subsequently, about 2 g of the photochromic curable composition obtained by the above method was applied to the surface of a lens substrate (CR39: acrylic resin plastic lens; refractive index of 1.50) by using the 1H-DX2 spin coater of MIKASA Co., Ltd. This coated lens was irradiated with light of a metal halide lamp having an output of 120 mW/cm² in a nitrogen gas atmosphere for 3 minutes to cure the photochromic curable composition so as to produce a photochromic plastic lens which is an optical article coated with a polymer film containing the chromene compound dispersed therein (thickness of polymer film: 40 µm).

The following photochromic properties of the obtained photochromic plastic lens were evaluated. The results are shown in Table 3.
[1] Maximum absorption wavelength (λmax) : This is the maximum absorption wavelength after color development obtained by means of the spectrophotometer (MCPD3000 instantaneous multi-channel photodetector) of Otsuka Electronics Co., Ltd. and used as an index of color at the time of color development.
[2] Color optical density (A₀) : This is the difference between absorbance {ε(120) after 120 seconds of exposure and ε(0) under no exposure at the above maximum absorption wavelength and used as an index of color optical density. As this value becomes larger, the photochromic properties become better.
[3] Double peak characteristic (A_{Y}/A_{B}) : This is the ratio of color optical density (A_{Y}: value of λₘₐₓ) at a yellow range (430 to 530 nm) and color optical density (A_{B}: value of λₘₐₓ) at a blue range (550 to 650 nm) and used as an index of double peak characteristic.
[4] Fading half period [τ1/2(sec.)]: time required for the reduction of the absorbance at the above maximum absorption wavelength of a sample to 1/2 of {ε(120)-ε(0)} when exposure is stopped after 120 seconds of exposure and used as an index of fading speed. As this time becomes shorter, the fading speed becomes higher.
[5] Absorption end {λ₀} : After the photochromic plastic lens obtained under the above conditions is used as a sample and kept in the dark for one day, the transmittance (T%) at 300 to 800 nm of the sample is measured with an ultraviolet visible spectrophotometer (UV-2550 of Shimadzu Corporation) at room temperature. A tangent line is drawn on the obtained transmittance curve to ensure that the transmittance (T%) of the obtained transmittance curve passes a point of 50 % so as to obtain a wavelength at which the transmittance (T%) of the tangent line becomes 0 as the absorption end (absorption end of the spectrum) and used as an index of initial coloration. For example, in an optical article such as a spectacle lens, as this value becomes smaller, the initial coloration becomes weaker and the transparency under no irradiation becomes higher.
[6] Thermochromism {T₀}: The photochromic plastic lens obtained under the above conditions is used as a sample and its transmittance (T%) at 300 to 800 nm is measured with an ultraviolet visible spectrophotometer (UV-2550 of Shimadzu Corporation) at room temperature. This is a transmittance at a wavelength at which the transmittance at 430 to 650 nm becomes minimal and used as an index of initial coloration. As this value becomes larger, the initial coloration becomes weaker and the transparency under no irradiation becomes higher.
[7] Residual rate (A₅₀/A₀ x 100) : The deterioration promotion test of the obtained photochromic plastic lens is carried out by using the X25 xenon weather meter of Suga Test Instruments Co., Ltd. for 50 hours. Thereafter, the above color optical density is evaluated before and after the test, the color optical density (A₀) before the test and the color optical density (A₅₀) after the test are measured, and the ratio (A₅₀/A₀) of these values is taken as residual rate and used as an index of color development durability. As the residual rate becomes higher, the color development durability becomes higher.

### Examples 5 and 6

The characteristic properties of photochromic plastic lenses were evaluated in the same manner as in Example 4 except that the compounds obtained in Examples 2 and 3 were used as the chromene compounds. The results are shown in Table 3. In Table 3, the compound number corresponds to Example number (for example, the compound No.1 is the chromene compound of Example 1).

**Table 3**

| Example No. | Compound No. | λ max | Color optical density | Double peak characteristic | Fading half period | Initial coloration (absorption | Initial coloration (thermochromism) | Residual rate |
|---|---|---|---|---|---|---|---|---|
| | | (nm) | A₀ | A_{Y}/A_{B} | τ1/2 (sec.) | (nm) | (%) | (A₅₀/A₀)×100 |
| 4 | 1 | 446 | 0.35 | 1.25 | 62 | 405 | 85 | 88 |
| | | 567 | 0.28 | | 59 | | 86 | 89 |
| 5 | 2 | 462 | 0.33 | 1.22 | 59 | 405 | 87 | 83 |
| | | 563 | 0.27 | | 58 | | 88 | 84 |
| 6 | 3 | 448 | 0.26 | 1.24 | 89 | 407 | 86 | 83 |
| | | 569 | 0.21 | | 89 | | 87 | 84 |

### Comparative Examples 1 to 5

For comparison, the operation of Example 4 was repeated by using the compound of the following formula (A) (Comparative Example 1), the compound of the following formula (B) (Comparative Example 2), the compound of the following formula (C) (Comparative Example 3), the compound of the following formula (D) (Comparative Example 4) and the compound of the following formula (E) (Comparative Example 5). The chromene compounds used in these comparative examples are given below.

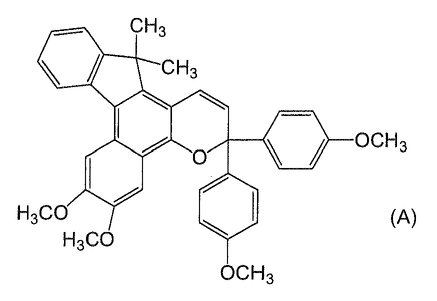

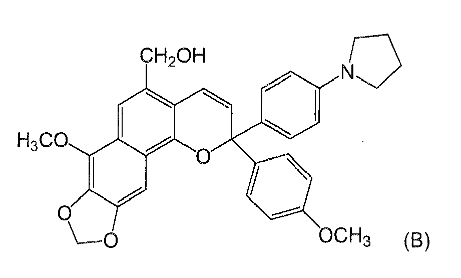

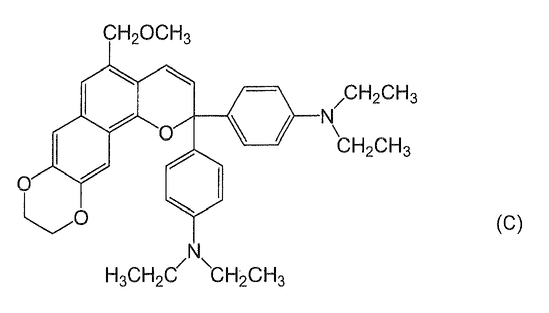

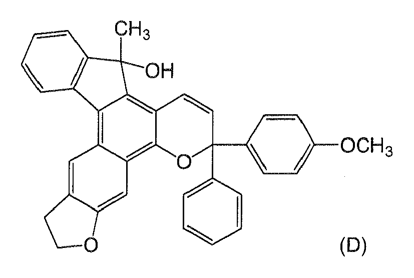

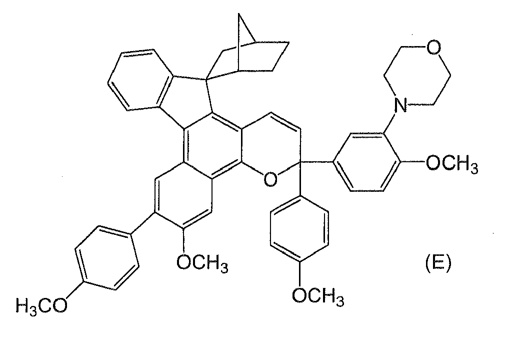

Photochromic plastic lenses were obtained by using the above chromene compounds and their photochromic properties were evaluated in the same manner as in Example 4. The results are shown in Table 4.

**Table 4**

| Comparative Example No. | Compound No. | λ max | color optical density | Double peak characteristic | Fading half period | Initial coloration (absorption end) | Initial coloration (thermochromism) | Residual rate |
|---|---|---|---|---|---|---|---|---|
| | | (nm) | A₀ | A_{Y}/A_{B} | τ1/2 (sec) | (nm) | (%) | (A₅₀/A₀) ×100 |
| 1 | A | 457 | 0.69 | 1.56 | 195 | 397 | 67 | 76 |
| | | 574 | 0.45 | | 196 | | 75 | 77 |
| 2 | B | 475 | 0.26 | 0.80 | 140 | 404 | 77 | 69 |
| | | 585 | 0.32 | | 140 | | 77 | 69 |
| 3 | C | 475 | 0.19 | 0.53 | 82 | 404 | 68 | 65 |
| | | 593 | 0.33 | | 82 | | 65 | 65 |
| 4 | D | 455 | 0.30 | 0.94 | 83 | 410 | 77 | 35 |
| | | 576 | 0.32 | | 83 | | 78 | 35 |
| 5 | E | 458 | 0.44 | 1.20 | 68 | 422 | 84 | 85 |
| | | 568 | 0.37 | | 68 | | 86 | 84 |

In Comparative Example 1, although the color optical density and double peak characteristic of the lens are satisfactory, the initial coloration due to thermochromism is large and the fading speed is low. In Comparative Examples 2, 3 and 4, the double peak characteristic is low, which is not preferred in terms of color control when a photochromic plastic lens developing a color of a neutral tint is to be manufactured. In Comparative Example 5, although the color optical density and double peak characteristic of the lens are satisfactory, the initial coloration is large as the absorption end goes beyond 420 nm into a visible range.

In contrast to this, in Examples in which the chromene compound of the present invention is used, as compared with Comparative Example 1, the initial coloration due to thermochromism is little and the fading speed is high. As compared with Comparative Examples 2, 3 and 4, the double peak characteristic is high. Since the absorption end is at a short wavelength as compared with Comparative Example 5, the initial coloration is little. Further, the chromene compounds of the present invention are all satisfactory in terms of durability.

### Examples 7 to 28

Chromene compounds shown in Table 5 were synthesized in the same manner as in Example 1. When the structures of the obtained chromene compounds were analyzed in the same manner as in Example 1, it was confirmed that they were compounds represented by the structural formulas shown in Table 5. Table 6 shows the elemental analysis values and ¹H-NMR spectral values of the chromene compounds obtained in Examples. In Table 6, the compound Nos. 7 to 28 are chromene compounds obtained in Examples 7 to 28, respectively.

**Table 5**

| Ex. No. | Raw materials | | Product | Yield rate (%) |
|---|---|---|---|---|
| | Naphthol compound | Propargyl alcohol compound | | |
| 7 | 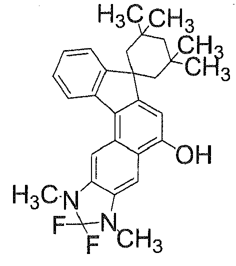 | 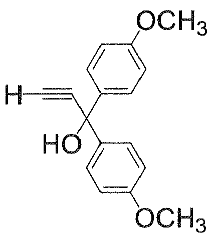 | 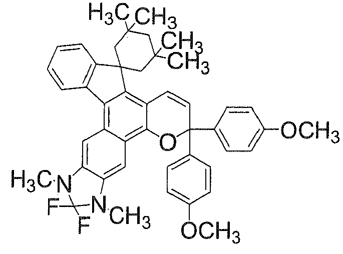 | 69 |
| 8 | 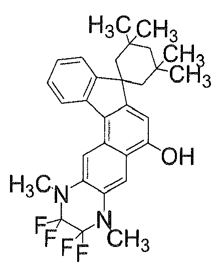 | 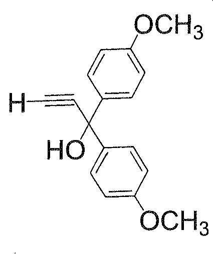 | 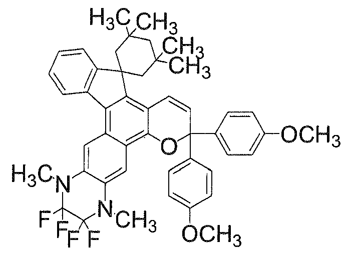 | 60 |
| 9 | 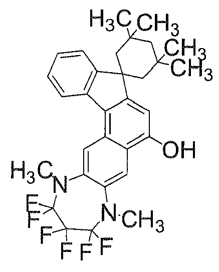 | 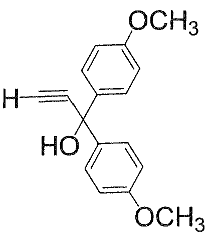 | 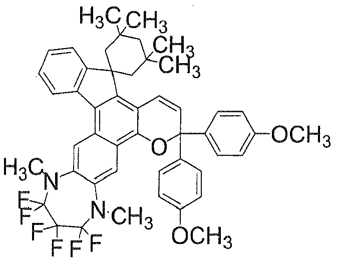 | 59 |
| 10 | 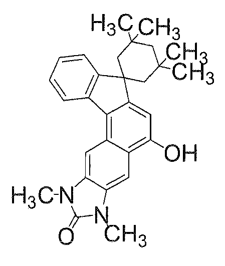 | 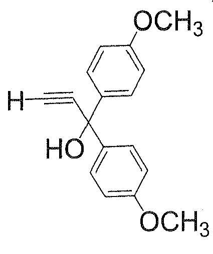 | 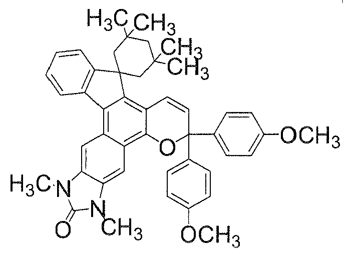 | 64 |
| 11 | 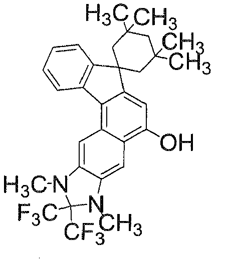 | 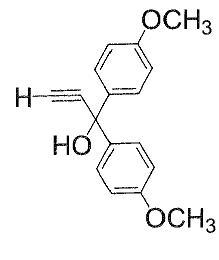 | 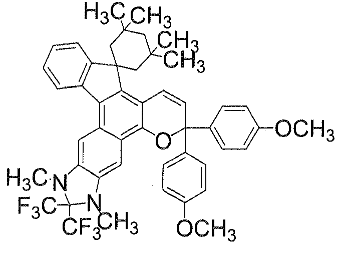 | 66 |
| 12 | 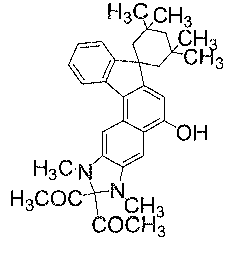 | 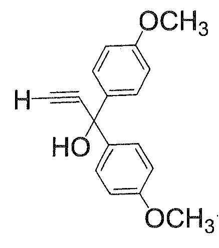 | 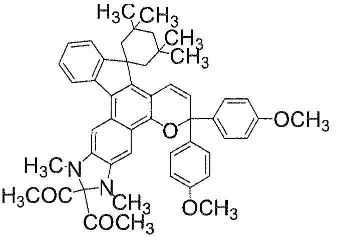 | 61 |
| 13 | 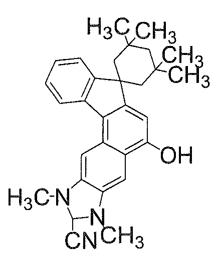 | 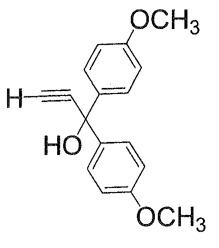 | 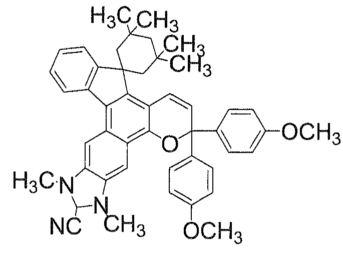 | 65 |
| 14 | 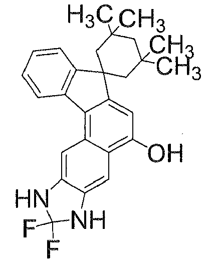 | 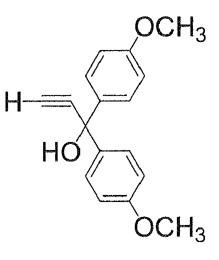 | 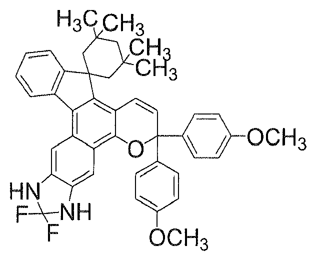 | 56 |
| 15 | 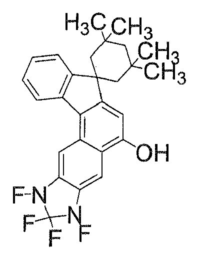 | 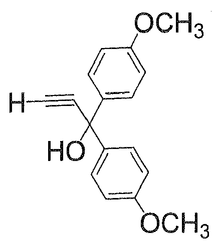 | 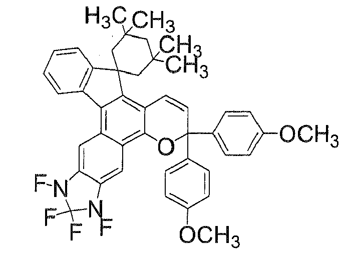 | 65 |
| 16 | 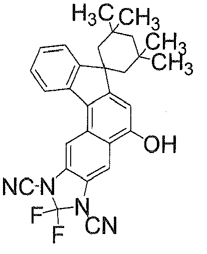 | 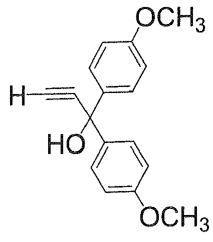 | 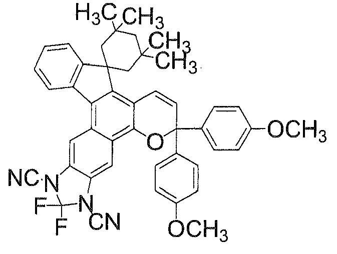 | 62 |
| 17 | 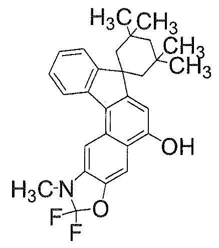 | 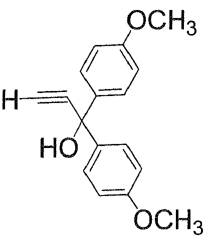 | 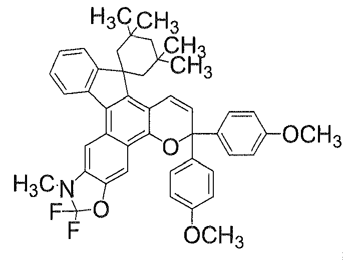 | 61 |
| 18 | 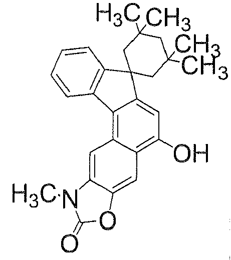 | 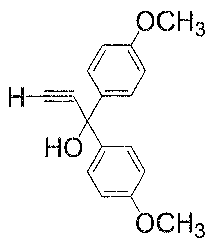 | 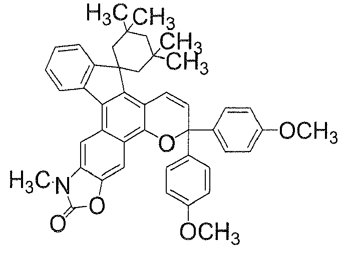 | 60 |
| 19 | 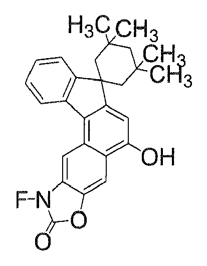 | 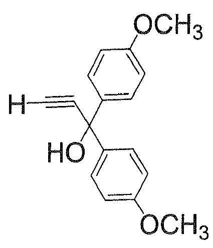 | 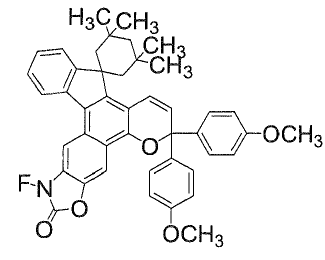 | 61 |
| 20 | 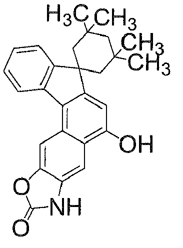 | 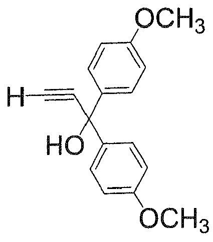 | 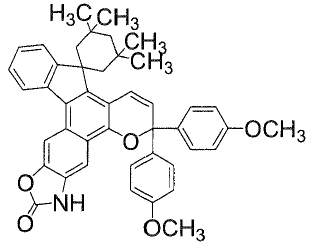 | 62 |
| 21 | 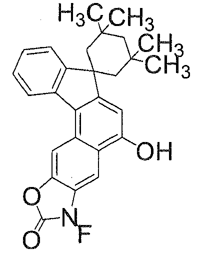 | 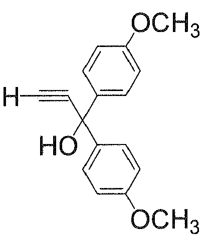 | 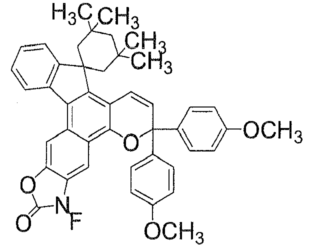 | 58 |
| 22 | 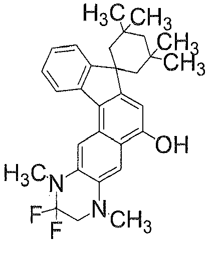 | 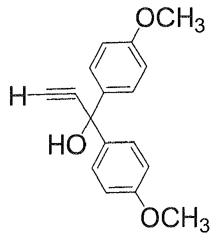 | 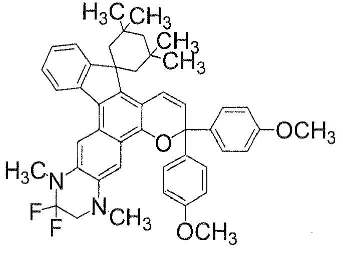 | 62 |
| 23 | 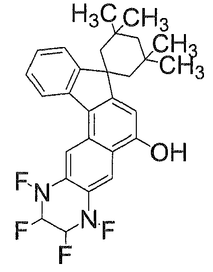 | 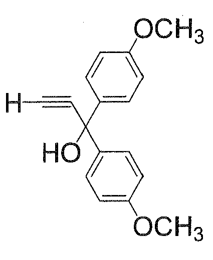 | 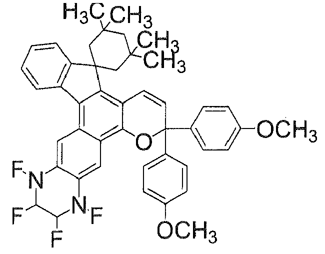 | 64 |
| 24 | 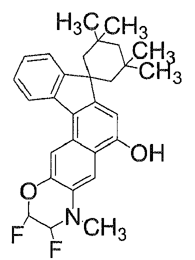 | 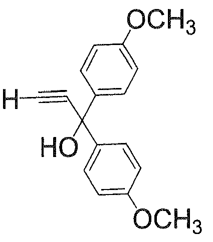 | 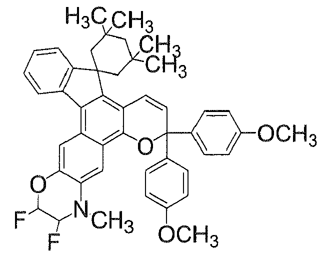 | 58 |
| 25 | 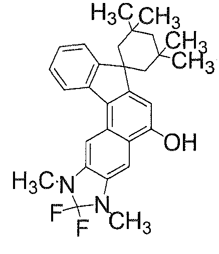 | 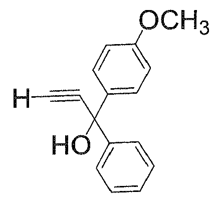 | 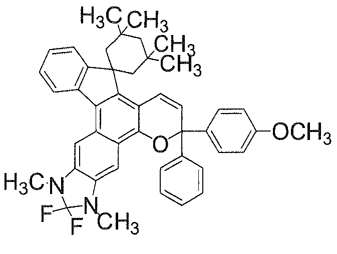 | 59 |
| 26 | 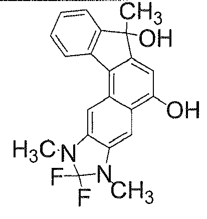 | 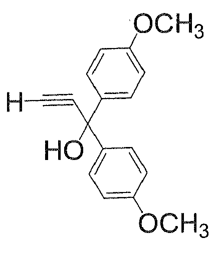 | 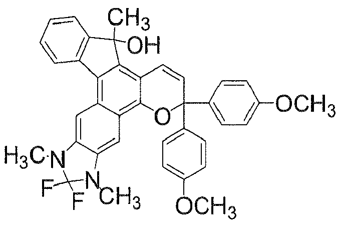 | 60 |
| 27 | 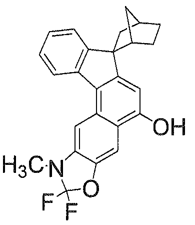 | 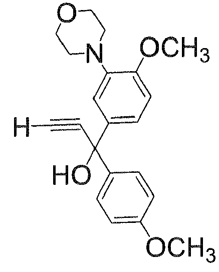 | 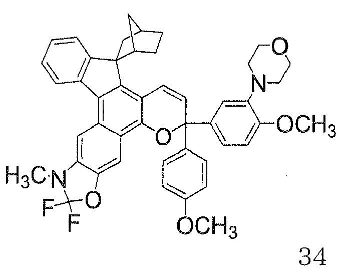 | 64 |
| 28 | 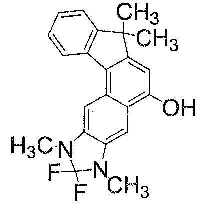 | 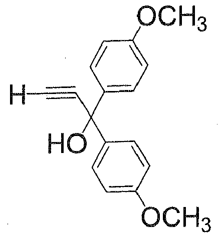 | 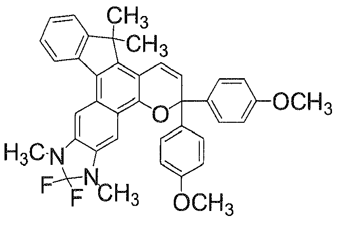 | 60 |

| | | | | |
|---|---|---|---|---|
| Ex.No.: Example Number | | | | |

**Table 6**

| Compound No. | Elemental analysis values | | | | | | ¹H-NMR (NMR) | |
|---|---|---|---|---|---|---|---|---|
| | Experimental values | | | Calculated values | | | | |
| | C | H | N | C | H | N | | |
| 7 | 77.45 | 6.42 | 4.02 | 77.50 | 6.50 | 3.93 | δ0.5-4.5 δ0.5-4.5 | 16H 30H |
| 8 | 74.09 | 6.01 | 3.77 | 74.00 | 6.08 | 3.67 | δ0.5-4.5 δ0.5-4.5 | 16H 30H |
| 9 | 70.90 | 5.70 | 3.54 | 70.92 | 5.70 | 3.45 | δ.0-9.0 δ0.5-4.5 | 16H 30H |
| 10 | 79.91 | 6.68 | 4.07 | 79.97 | 6.71 | 4.05 | δ5.0-9.0 δ0.5-4.5 | 16H 30H |
| 11 | 70.99 | 5.77 | 3.44 | 70.92 | 5.70 | 3.45 | δ5.0-9.0 δ0.5-4.5 | 16H 30H |
| 12 | 78.90 | 6.99 | 3.62 | 78.92 | 6.89 | 3.68 | δ5.0-9.0 δ0.5-4.5 | 16H 36H |
| 13 | 80.44 | 6.77 | 6.03 | 80.43 | 6.75 | 5.99 | δ5.0-9.0 δ0.5-4.5 | 16H 31H |
| 14 | 77.13 | 6.22 | 4.11 | 77.17 | 6.18 | 4.09 | δ5.0-9.0 δ0.5-4.5 | 16H 26H |
| 15 | 73.33 | 5.62 | 3.92 | 73.32 | 5.59 | 3.89 | δ0.5-9.0 δ0.5-4.5 | 16H 14H |
| 16 | 75.21 | 5.55 | 7.65 | 75.19 | 5.49 | 7.62 | δ5.0-9.0 δ0.5-4.5 | 16H 24H |
| 17 | 77.32 | 6.20 | 1.95 | 77.23 | 6.19 | 2.00 | δ5.0-9.0 δ0.5-4.5 | 16H 27H |
| 18 | 79.78 | 6.35 | 2.03 | 79.74 | 6.39 | 2.07 | δ5.0-9.0 δ0.5-4.5 | 16H 27H |
| 19 | 77.55 | 5.88 | 2.04 | 77.51 | 5.91 | 2.05 | δ5.0-9.0 δ0.5-4.5 | 16h 24H |
| 20 | 79.66 | 6.22 | 2.07 | 79.61 | 6.23 | 2.11 | δ5.0-9.0 δ0.5-4.5 | 16H 25H |
| 21 | 77.65 | 5.81 | 2.00 | 77.51 | 5.91 | 2.05 | δ5.0-9.0 δ0.5-4.5 | 16H 14H |
| 22 | 77.56 | 6.66 | 3.82 | 77.66 | 6.66 | 3.85 | δ5.0-9.0 δ0.5-4-5 | 16H 32H |
| 23 | 73.56 | 5.77 | 3.90 | 3.55 | 5.76 | 3.81 | δ5.0-9.0 δ0.5-4.5 | 16H 26H |
| 24 | 77.45 | 6.33 | 1.99 | 77.40 | 6.35 | 1.96 | δ5.0-9.0 δ0.5-4.5 | 16H 29H |
| 25 | 79.20 | 6.44 | 4.11 | 79.15 | 6.49 | 4.10 | δ5.0-9.0 δ0.5-4.5 | 17H 27H |
| 26 | 73.80 | 5.32 | 4.47 | 73.77 | 5.21 | 4.53 | δ5.0-9.0 δ0.5-4.5 | 16H 16H |
| 27 | 74.56 | 5.44 | 3.83 | 74.58 | 5.41 | 3.78 | δ5.0-9.0 δ0.5-4.5 | 15H 27H |
| 28 | 75.99 | 5.51 | 4.55 | 75.96 | 5.56 | 4.54 | δ5.0-9.0 δ0.5-4.5 | 16H 18H |

### Examples 29 to 50

Photochromic plastic lenses were manufactured and their characteristic properties were evaluated in the same manner as in Example 4 except that the compounds obtained in Examples 7 to 28 were used as chromene compounds. The results are shown in Table 7. In Table 7, the compound Nos. 7 to 28 are chromene compounds obtained in Examples 7 to 28, respectively.

**Table 7**

| Example No. | Compound No. | λ max | Compound No. | Double peak characteristic | Fading half period | Initial coloration (absorption end) | Initial coloration (thermochromism) | Residual rate |
|---|---|---|---|---|---|---|---|---|
| | | (nm) | A₀ | A_{Y}/A_{B} | τ1/2 (sec) | (nm) | (%) | (A_{50/}A₀) X100 |
| 29 | 7 | 475 | 0.50 | 1.62 | 72 | 413 | 84 | 84 |
| | | 564 | 0.31 | | 73 | | 84 | 85 |
| 30 | 8 | 450 | 0.50 | 1.56 | 91 | 407 | 84 | 83 |
| | | 571 | 0.32 | | 91 | | 84 | 84 |
| 31 | 9 | 460 | 0.50 | 1.47 | 106 | 410 | 84 | 83 |
| | | 567 | 0.34 | | 106 | | 84 | 83 |
| 32 | 10 | 470 | 0.49 | 1.53 | 68 | 410 | 85 | 80 |
| | | 567 | 0.32 | | 69 | | 86 | 80 |
| 33 | 11 | 443 | 0.42 | 1.17 | 50 | 403 | 88 | 82 |
| | | 560 | 0.36 | | 50 | | 88 | 82 |
| 34 | 12 | 439 | 0.41 | 1.08 | 44 | 400 | 88 | 80 |
| | | 556 | 0.38 | | 44 | | 88 | 80 |
| 35 | 13 | 444 | 0.43 | 1.16 | 57 | 404 | 85 | 86 |
| | | 556 | 0.37 | | 58 | | 86 | 86 |
| 36 | 14 | 473 | 0.49 | 1.53 | 70 | 411 | 85 | 82 |
| | | 565 | 0.32 | | 70 | | 85 | 82 |
| 37 | 15 | 468 | 0.43 | 1.34 | 53 | 404 | 88 | 86 |
| | | 566 | 0.32 | | 53 | | 89 | 86 |
| 38 | 16 | 436 | 0.39 | 1.00 | 46 | 401 | 87 | 80 |
| | | 552 | 0.39 | | 46 | | 87 | 80 |
| 39 | 17 | 465 | 0.44 | 1.47 | 59 | 412 | 86 | 84 |
| | | 566 | 0.30 | | 59 | | 86 | 84 |
| 40 | 18 | 465 | 0.40 | 1.25 | 61 | 409 | 86 | 83 |
| | | 570 | 0.32 | | 61 | | 86 | 83 |
| 41 | 19 | 463 | 0.40 | 18 | 56 | 410 | 87 | 83 |
| | | 565 | 0.34 | | 56 | | 87 | 83 |
| 42 | 20 | 462 | 0.40 | 1.21 | 60 | 405 | 85 | 80 |
| | | 564 | 0.33 | | 61 | | 86 | 80 |
| 43 | 21 | 459 | 0.40 | 1.18 | 55 | 403 | 87 | 81 |
| | | 561 | 0.34 | | 55 | | 88 | 82 |
| 44 | 22 | 456 | 0.51 | 1.65 | 96 | 406 | 83 | 83 |
| | | 573 | 0.31 | | 96 | | 83 | 83 |
| 45 | 23 | 447 | 0.43 | 1.30 | 89 | 403 | 88 | 85 |
| | | 570 | 0.33 | | 89 | | 88 | 85 |
| 46 | 24 | 459 | 0.42 | 1.40 | 65 | 408 | 86 | 84 |
| | | 561 | 0.30 | | 66 | | 87 | 84 |
| 47 | 25 | 469 | 0.54 | 1.69 | 115 | 413 | 85 | 78 |
| | | 559 | 0.32 | | 116 | | 85 | 78 |
| 48 | 26 | 476 | 0.53 | 1.61 | 90 | 413 | 80 | 78 |
| | | 565 | 0.33 | | 90 | | 80 | 78 |
| 49 | 27 | 475 | 0.45 | 1.73 | 80 | 410 | 85 | 81 |
| | | 568 | 0.26 | | 80 | | 86 | 81 |
| 50 | 28 | 476 | 0.65 | 1.63 | 133 | 413 | 78 | 80 |
| | | 565 | 0.40 | | 133 | | 79 | 80 |

Examples of the naphthol compound are given below.

### Example 51

32. 9 g (178.5 mmol) of a benzene derivative represented by the following formula (18) and 15 g of Wakogel C-300 (of Wako Pure Chemical Industries, Ltd.) were dissolved in 2,000 ml of dichloromethane, the resulting solution was cooled to -15°C, and 31.3 g (174.9 mmol) of N-bromosuccinimide was added and stirred for 12 hours. After the reaction, the reaction product was washed in water, the solvent was removed, and the obtained product was purified by column chromatography to obtain a compound represented by the following formula (19) as 44.9 g (171.4 mmol, yield rate of 96 %) of orange oil.

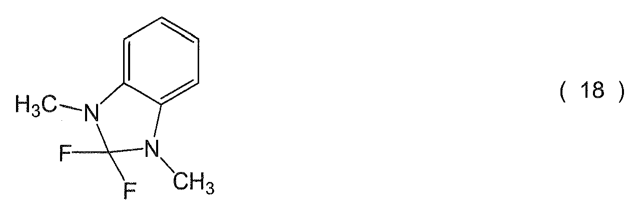

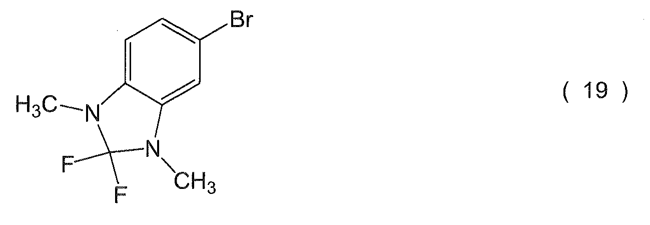

4.6 g (188.5 mmol) of magnesium was added to 200 ml of tetrahydrofuran and heated to 55°C. A tetrahydrofuran (200 ml) solution of the compound of the above formula (19) was added dropwise to the above solution to prepare a Grignard reagent. The obtained Grignard reagent was cooled to -78°C, and a tetrahydrofuran (200 ml) solution of 26.5 g (188. 5 mmol) of benzoyl chloride was added dropwise to the reagent. After addition, the resulting solution was heated to room temperature and stirred for 3 hours. After the reaction, the reaction product was washed in water, the solvent was removed, and the obtained product was purified by recrystallization with methanol to obtain a compound represented by the following formula (20) as 37.0 g (128.6 mmol, yield rate of 75 %) of a yellow solid.

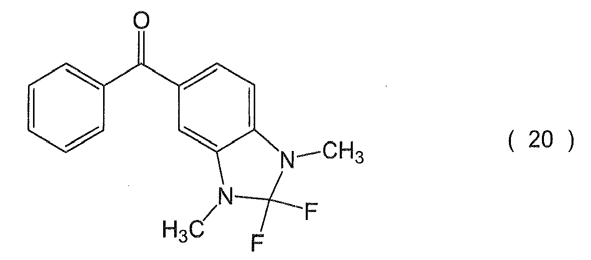

The compound of the above formula (20) and 25.8 g (147.9 mmol) of diethyl succinate were dissolved in 250 ml of tetrahydrofuran and heated to 55°C. A tetrahydrofuran solution (250 ml) of 16.6 g (147.9 mmol) of potassium-t-butoxide was added dropwise to this solution and stirred for 1 hour. After the reaction, the reaction product was washed with concentrated hydrochloric acid and then with water, and the solvent was removed to obtain a compound represented by the following formula (21) as 53.5 g (128.6 mmol, yield rate of 100 %) of orange oil.

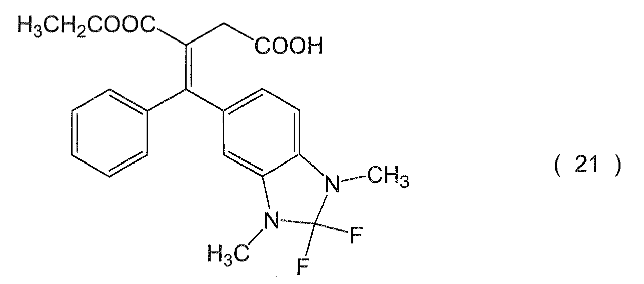

The compound of the above formula (21), 11.6 g (141.5 mmol) of sodium acetate and 72.2 g (707.5 mmol) of acetic anhydride were dissolved in 180 ml of toluene and refluxed for 3 hours. After the reaction, the reaction product was washed in water, the solvent was removed, and the obtained product was purified by recrystallization with methanol to obtain a compound represented by the following formula (22) as 17.0 g (38.6 mmol, yield rate of 30 %) of an orange solid.

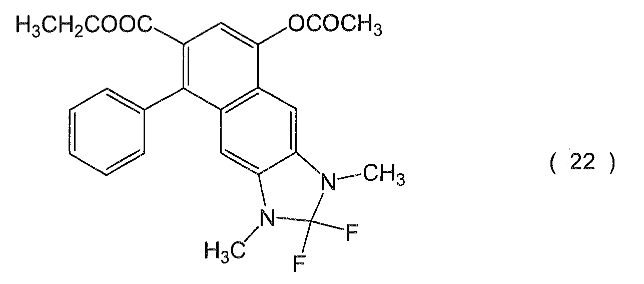

The compound of the above formula (22) was dispersed into 80 ml of methanol. 300 ml of an aqueous solution of 27.8 g (694.8 mmol) of sodium hydroxide was added to this solution and refluxed for 3 hours. After the reaction, the reaction product was washed with concentrated hydrochloric acid and then with water, the solvent was removed, and the obtained product was purified by reslurrying with toluene to obtain a compound represented by the following formula (23) as 12.9 g (34.7 mmol, yield rate of 90 %) of a yellow solid.

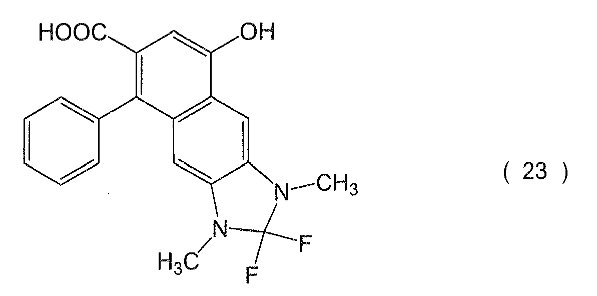

The compound of the above formula (23) and 9.7 g (76.3 mmol) of benzyl chloride were dissolved in 160 ml of N,N-dimethylformamide. 12.0 g (86.8 mmol) of potassium carbonate was added to this solution, heated to 60°C and stirred for 3 hours. After the reaction, the reaction product was washed in water, and the solvent was removed to obtain a compound represented by the following formula (24) as 18.7 g (34.0 mmol, yield rate of 98 %) of yellow oil.

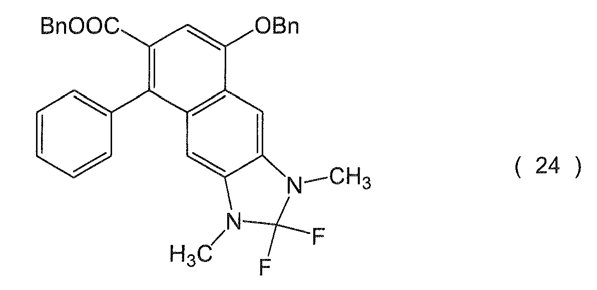

The compound of the above formula (24) was dispersed into 150 ml of isopropyl alcohol. 120 ml of an aqueous solution of 20.4 g (510.0 mmol) of sodium hydroxide was added to this solution and refluxed for 3 hours. After the reaction, the reaction product was washed with concentrated hydrochloric acid and then with water, the solvent was removed, and the obtained product was purified by reslurrying with toluene to obtain a compound represented by the following formula (25) as 13.6 g (29.6 mmol, yield rate of 87 %) of a yellow solid.

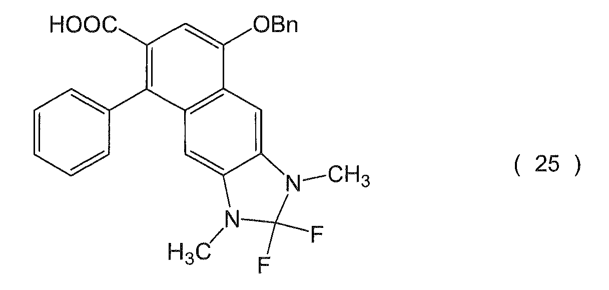

The compound of the above formula (25) was dispersed into 350 ml of toluene. 9.0 g (88.8 mmol) of triethylamine and 10.6 g (38.5 mmol) of diphenylphosphorylazide were added to this solution and stirred at room temperature for 2 hours. 6.8 g (148.0 mmol) of ethanol was added to this solution to carry out a reaction at 70°C for 2 hours. Thereafter, 100 ml of ethanol was added to this solution, and then 16.6 g (296.0 mmol) of potassium hydroxide was added and refluxed for 5 hours. After the reaction, ethanol was distilled off at normal pressure, tetrahydrofuran was added, the solution was washed in water, and the solvent was removed to obtain a compound represented by the following formula (26) as 12.8 g (29.6 mmol, yield rate of 100 %) of a yellow solid.

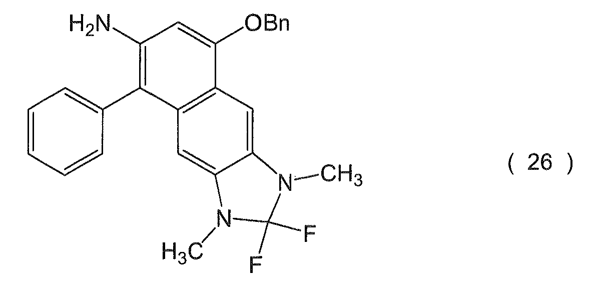

The compound of the above formula (26) was dispersed into 500 ml of acetonitrile, and 93.5 g (148.0 mmol) of a 6 % hydrochloric acid aqueous solution was added to the dispersion and cooled to 0 to 5°C. 18.4 g (88.8 mmol) of a 33 % sodium nitrite aqueous solution was added to this solution and stirred for 30 minutes. 51.5 g (148. mmol) of a 50 % potassium iodide aqueous solution was added to this solution and stirred at room temperature for 5 hours. After the reaction, toluene was added, the reaction product was washed in water, the solvent was removed, and the obtained product was purified by column chromatography to obtain a compound represented by the following formula (27) as 11.2 g (20.7 mmol, yield rate of 70 %) of a yellow solid.

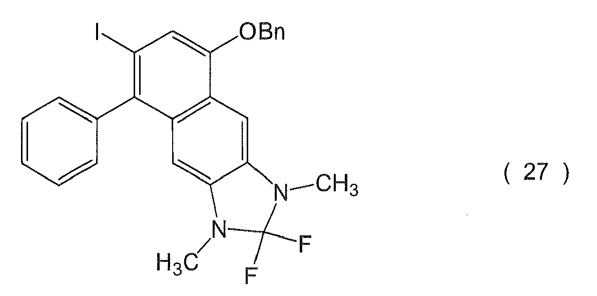

The compound of the above formula (27) was dispersed into 600 ml of toluene and cooled to -30°C. 15.6 ml (24.9 mmol) of n-butyl lithium (1.6M hexane solution) was added dropwise to this solution and stirred for 30 minutes. 8.0 ml of a toluene solution of 4.0 g (25.9 mmol) of 3,3,5,5-tetramethylcyclohexanone was added dropwise to this solution and stirred at 0 °C for 3 hours. After the reaction, toluene was added, the reaction product was washed in water, the solvent was removed, and the obtained product was purified by reslurrying with methanol to obtain a compound represented by the following formula (28) as 7.7 g (13.5 mmol, yield rate of 65 %) of a yellow solid.

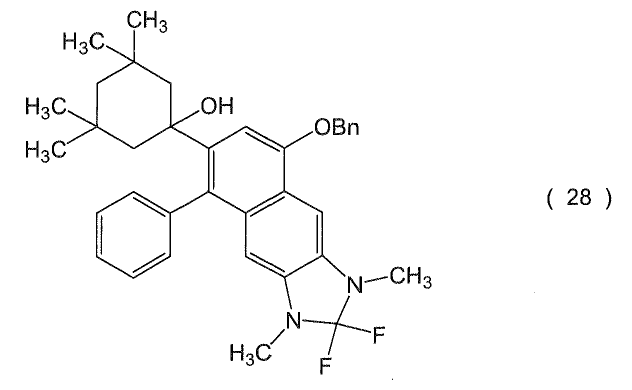

The compound of the above formula (28) was dissolved in 200 ml of tetrahydrofuran, and 3.4 g (54.0 mmol) of ammonium formate and 3.8 g of 5 % palladium carbon were added and stirred at room temperature for 8 hours. After the reaction, toluene was added, the reaction product was washed in water, the solvent was removed, and the obtained product was purified by reslurrying with toluene to obtain a compound represented by the following formula (29) as 5.8 g (12.2 mmol, yield rate of 90 %) of a yellow solid.

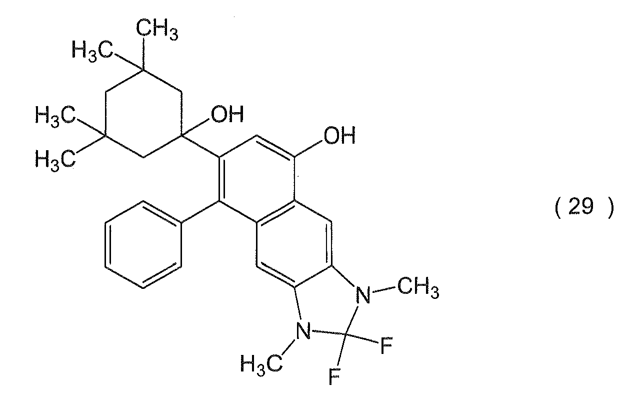

The compound of the above formula (29) was dissolved in 150 ml of toluene and heated to 90°C. 7.0 g (36.6 mmol) of p-toluensulfonic acid was added to this solution and refluxed for 3 hours. After the reaction, the reaction product was washed in water, and the solvent was removed to obtain a naphthol compound represented by the following formula (30) as 4.3 g (9.2 mmol, yield rate of 75 %) of a yellow solid.

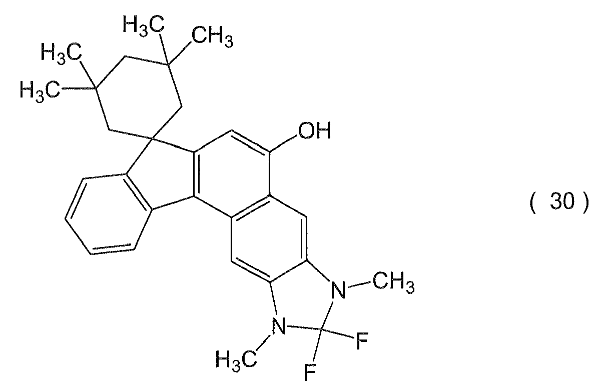

The elemental analysis values of this product were 75.21 % of C, 7.04 % of H and 5.99 % of N which were almost equal to the calculated values of C₂₉H₃₂F₂N₂0 (C: 75.30 %, H: 6.97 %, N: 6.06 %).

When the proton nuclear magnetic resonance spectrum was measured, it showed a 24H peak based on an alkyl group at δ of around 0.5 to 4.5 ppm and 8H peaks based on a hydroxyl group and an aromatic proton at δ of around 5.0 to 9.0 ppm

Further, when the ¹³C-nuclear magnetic resonance spectrum was measured, it showed a peak based on the carbon of an aromatic ring at δ of around 110 to 160 ppm and a peak based on the carbon of an alkyl group at δ of around 20 to 80 ppm.

It was confirmed from these results that the isolated product was a naphthol compound represented by the above formula (30).

This compound is a naphthol compound used in the above Example 7.

### Examples 52 to 74

Naphthol compounds shown in the table were synthesized in the same manner as in Example 51. When the structures of the obtained products were analyzed by using the same structure confirming means as in Example 51, it was confirmed that they were naphthol compounds used in Examples shown in Table 8. Table 8 shows the elemental analysis values, calculated values obtained from the structural formulas of the compounds and characteristic ¹H-NMR spectra of these compounds.

**Table 8**

| Example No. | Examples of chromene compounds No.* | Elemental analysis values | | | | | | ¹H-NMR (NMR) |
|---|---|---|---|---|---|---|---|---|
| | | Experimental values | | | Calculated values | | | |
| | | C | H | N | C | H | N | |
| 52 | 1 | 72.88 | 6.77 | 5.62 | 72.85 | 6.73 | 5.66 | δ5-0-9.0 8H δ0.5-4.5 25H |
| 53 | 2 | 69.44 | 5.11 | 3.02 | 69.37 | 5.17 | 3.00 | δ5.0-9.0 7H δ0.5-4.5 17H |
| 54 | 3 | 66.66 | 5.96 | 4.82 | 66.65 | 5.94 | 4.86 | δ5.0-9.0 8H δ0.5-4.5 26H |
| 55 | 8 | 70.33 | 6.34 | 5.54 | 70.30 | 6.29 | 5.47 | δ5.0-9.0 8H δ0.5-4.5 24H |
| 56 | 9 | 66.21 | 5.77 | 5.00 | 66.18 | 5.73 | 4.98 | δ5.0-9.0 8H δ0.5-4.5 24H |
| 57 | 10 | 79.04 | 7.33 | 6.27 | 79.06 | 7.32 | 6.36 | δ5.0-9.0 24H δ0.5-4.5 24H |
| 58 | 11 | 66.21 | 5.67 | 5.02 | 66.18 | 5.73 | 4.98 | δ5.0-9.0 8H δ0.5-4.5 24H |
| 59 | 12 | 77.56 | 7.49 | 5.54 | 77.61 | 7.50 | 5.49 | δ5-0-9.0 8H δ0.5-4.5 30H |
| 60 | 13 | 79.74 | 7.45 | 9.32 | 79.79 | 7.37 | 9.30 | δ5.0-9.0 8H δ0.5-4.5 25H |
| 61 | 14 | 74.56 | 6.55 | 6.46 | 74.63 | 6.50 | 6.45 | δ5.0-9.0 8H δ0.5-4.5 20H |
| 62 | 15 | 68.91 | 5.61 | 5.92 | 68.92 | 5.57 | 5.95 | δ5.0-9.0 8H δ0.5-4.5 18H |
| 63 | 16 | 71.84 | 5.44 | 11.50 | 71.88 | 5.41 | 11.56 | δ5.0-9.0 8H δ0.5-4.5 18H |
| 64 | 17 | 74.88 | 6.43 | 3.11 | 74.81 | 6.50 | 3.12 | δ5.0-9.0 8H δ0.5-4.5 21H |
| 65 | 18 | 78.61 | 6.83 | 3.33 | 78.66 | 6.84 | 3.28 | δ5.0-9.0 8H δ0.5-4.5 21H |
| 66 | 19 | 75.22 | 6.08 | 3.22 | 75.15 | 6.07 | 3.25 | δ5.0-9.0 8H δ0.5-4.5 18H |
| 67 | 20 | 78.43 | 6.65 | 3.31 | 78.42 | 6.58 | 3.39 | δ5.0-9.0 8H δ0.5-4.5 19H |
| 68 | 21 | 75.12 | 6.09 | 3.33 | 75.15 | 6.07 | 3.25 | δ5.0-9.0 8H δ0.5-4.5 18H |
| 69 | 22 | 75.52 | 7.22 | 5.89 | 75.60 | 7.19 | 5.88 | δ5-0-9.0 8H δ0.5-4.5 26H |
| 70 | 23 | 69.45 | 5.81 | 5.79 | 69.41 | 5.82 | 5.78 | δ5.0-9.0 8H δ0.5-4.5 20H |
| 71 | 24 | 75.11 | 6.75 | 3.01 | 75.14 | 6.74 | 3.02 | δ5.0-9.0 8H δ0.5-4.5 23H |
| 72 | 26 | 68.55 | 4.93 | 7.53 | 68.47 | 4.93 | 7.60 | δ0.5-4.5 10H δ0.5-4.5 10H |
| 73 | 27 | 74.00 | 5.21 | 3.46 | 74.06 | 5.22 | 3.45 | δ5.0-9.0 8H δ0.5-4.5 13H |
| 74 | 28 | 72.17 | 5.44 | 7.66 | 72.12 | 5.50 | 7.65 | δ5.0-9.0 8H δ0.5-4.5 12H |

| | | | | | | | | |
|---|---|---|---|---|---|---|---|---|
| *Examples of chromene compounds produced by using the naphthol compounds of Examples | | | | | | | | |

### Effect of the Invention

Since the chromene compound of the present invention develops a color of a neutral tint by itself, it can be used alone and hardly undergoes a color change at the time of fading and a color change at the time of deterioration. Further, since the chromene compound has little initial coloration, high color optical density, high double peak characteristic and high fading speed, an extremely excellent photochromic lens can be obtained from the chromene compound. Therefore, color can be controlled by mixing it with another photochromic compound, and even when it is mixed with another photochromic compound, it can exhibit excellent photochromic properties.

## Claims

1. A chromene compound having a skeleton represented by the following formula (1): 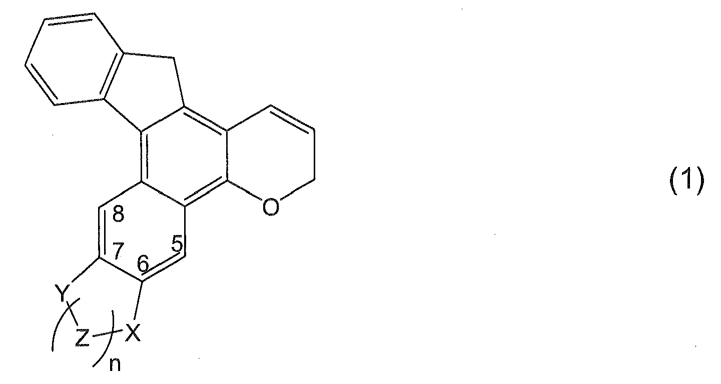 wherein Z is a group represented by any one of the following formulas: 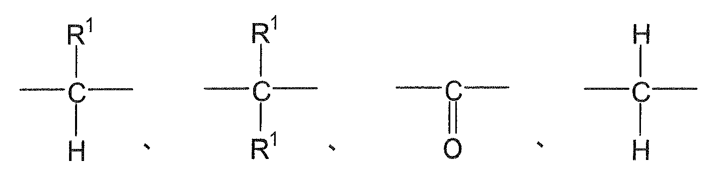 n is an integer of 1 to 3, when n is 2 or 3, Z's may be the same or different, with the proviso that when n is 1, Z cannot be -CH₂- and when n is 2 or 3, Z' s cannot be -CH₂- at the same time; R¹ is an electron absorbing group having a Hammett constant σₚ of more than 0, with the proviso that when there are a plurality of R¹'s, R¹'s may be the same or different; and X and Y are each independently an oxygen atom or a group represented by the following formula (2): 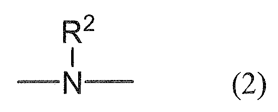 wherein R² is a hydrogen atom, hydroxyl group, alkyl group, haloalkyl group, alkenyl group, alkynyl group, cycloalkyl group, alkoxy group, aralkyl group, aralkoxy group, aryloxy group, aryl group, amino group, heterocyclic group having a nitrogen atom which is a cyclic membered hetero atom and is bonded to the nitrogen atom in the above formula (2), cyano group, nitro group, formyl group, hydroxycarbonyl group, alkylcarbonyl group, alkoxycarbonyl group or halogen atom, with the proviso that X and Y cannot be oxygen atoms at the same time.

2. The chromene compound according to claim 1 which is represented by the following formula (3): 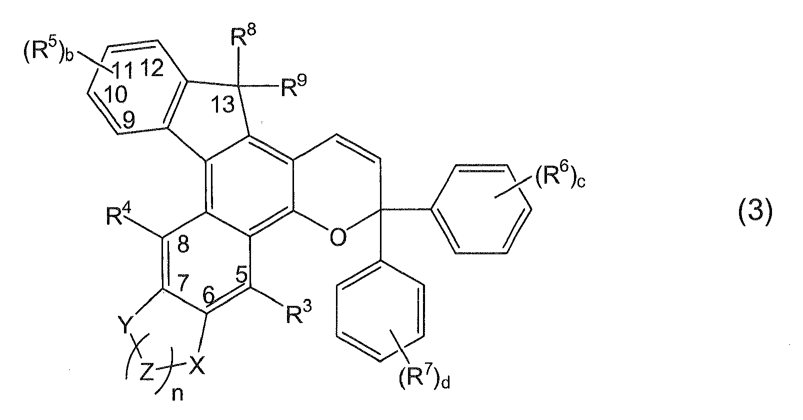 wherein Z, n, X and Y are as defined in the above formula (1) ; R³ and R⁴ are each independently a hydrogen atom, hydroxyl group, alkyl group, haloalkyl group, alkenyl group, alkynyl group, cycloalkyl group, alkoxy group, aralkyl group, aralkoxy group, aryloxy group, aryl group, amino group, heterocyclic group having a nitrogen atom which is a cyclic membered hetero atom and is bonded to the 5-position or 8-position carbon atom, cyano group, nitro group, formyl group, hydroxycarbonyl group, alkylcarbonyl group, alkoxycarbonyl group or halogen atom; R⁵, R⁶ and R⁷ are each independently a hydroxyl group, alkyl group, haloalkyl group, alkenyl group, alkynyl group, cycloalkyl group, alkoxy group, aralkyl group, aralkoxy group, aryloxy group, aryl group, amino group, heterocyclic group having a nitrogen atom which is a cyclic membered hetero atom and is bonded to the benzene ring bonded thereto, cyano group, nitro group, formyl group, hydroxycarbonyl group, alkylcarbonyl group, alkoxycarbonyl group or halogen atom; R⁸ and R⁹ are each independently a hydrogen atom, hydroxyl group, alkyl group, haloalkyl group, alkenyl group, alkynyl group, cycloalkyl group, alkoxy group, aralkyl group, aralkoxy group, aryloxy group, aryl group, amino group, heterocyclic group having a nitrogen atom which is a cyclic membered hetero atom and is bonded to the 13-position carbon atom, cyano group, nitro group, formyl group, hydroxycarbonyl group, alkylcarbonyl group, alkoxycarbonyl group or halogen atom, R⁸ and R⁹ may be bonded together to form a carbonyl group or aliphatic hydrocarbon ring together with the 13-position carbon atom, b is an integer of 0 to 4, c and d are each independently an integer of 0 to 5, with the proviso that when b is 2 or more, R⁵'s may be the same or different, and when c and d are each 2 or more, R⁶'s and R⁷'s may be the same or different.

3. The chromene compound according to claim 2, wherein, in the chromene compound represented by the above formula (3), R⁸ and R⁹ are bonded together to form an aliphatic hydrocarbon ring together with the 13-position carbon atom, and the aliphatic hydrocarbon ring has 4 to 20 cyclic membered carbon atoms and may have at least one substituent selected from the group consisting of alkyl group, haloalkyl group, cycloalkyl group, alkoxy group, amino group, aralkyl group, aryl group and halogen atom.

4. A photochromic curable composition comprising the chromene compound of any one of claims 1 to 3 and a polymerizable monomer.

5. A photochromic optical article having a polymer molded product comprising the chromene compound of any one of claims 1 to 3 dispersed therein as a constituent member.

6. An optical article comprising an optical substrate having a surface at least part of which is coated with a polymer film as a constituent part, wherein
the polymer film comprises the chromene compound of any one of claims 1 to 3 dispersed therein.

7. A naphthol compound represented by the following formula (4): 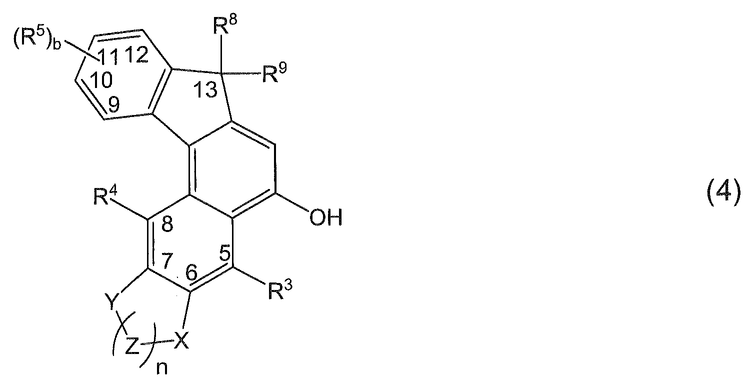 wherein R³, R⁴, R⁵, R⁸, R⁹, Z, X, Y, b and n are as defined in the above formula (3).
